(19) Europäisches Patentamt
European Patent Office
Office européen des brevets

(11) Publication number: 0 417 165 B1

(12) # EUROPEAN PATENT SPECIFICATION

(45) Date of publication of patent specification: **26.01.94**

(51) Int. Cl.[5]: **C07K 7/00**, C07K 7/06, A61K 37/02

(21) Application number: **89906526.2**

(22) Date of filing: **01.05.89**

(86) International application number: **PCT/US89/01829**

(87) International publication number: **WO 89/10933 (16.11.89 89/27)**

(54) **POLYPEPTIDE COMPOUNDS HAVING GROWTH HORMONE RELEASING ACTIVITY.**

(30) Priority: **11.05.88 US 192756**

(43) Date of publication of application: **20.03.91 Bulletin 91/12**

(45) Publication of the grant of the patent: **26.01.94 Bulletin 94/04**

(84) Designated Contracting States: **AT BE CH DE FR GB IT LI LU NL SE**

(56) References cited:
**EP-A- 0 018 072**
**EP-A- 0 083 864**
**WO-A-87/06835**
**WO-A-88/09780**

(73) Proprietor: **POLYGEN HOLDING CORPORATION**
**1201 North Market Street**
**Wilmington, Delaware 19899(US)**

(72) Inventor: **BOWERS, Cyril, Yarling**
**484 Audubon Street**
**New Orleans, LA 70118(US)**
Inventor: **MOMANY, Frank, Alden**
**1-1 Concord Green**
**Concord, MA 02154(US)**
Inventor: **CODY, Wayne, Livingston**
**Route 2, Box 65**
**Ringoes, NJ 08551(US)**
Inventor: **HUBBS, John, Clark**
**Route 10, Box 354A**
**Kingsport, TN 37664(US)**
Inventor: **FOSTER, Charles, Howard**
**1413 Dobyns Drive**
**Kingsport, TN 37664(US)**

(74) Representative: **Davies, Jonathan Mark et al**
**Reddie & Grose**
**16 Theobalds Road**
**London WC1X 8PL (GB)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to novel polypeptide compounds which promote the release of growth hormone when administered to animals. In another aspect, this invention relates to methods for promoting the release and elevation of growth hormone levels in animals by administration of specified growth hormone releasing polypeptide compounds thereto.

### Background of the Invention

It has been established in the scientific literature that the elevation of growth hormone (GH) levels in mammals upon administration of GH-releasing compounds can lead to enhanced body weight and to enhanced milk production if sufficiently elevated GH levels occur upon administration. Further, it is known that the elevation of growth hormone levels in mammals can be accomplished by application of known growth hormone releasing agents, such as the naturally occurring growth hormone releasing hormones.

The elevation of growth hormone levels in mammals can also be accomplished by application of growth hormone releasing peptides, some of which have been previously described, for example, by F. A. Momany in U.S. 4,223,019, U.S. 4,223,020, U.S. 4,223,021, U.S. 4,224,316, U.S. 4,226,857, U.S. 4,228,155, U.S. 4,228,156, U.S. 4,228,157, U.S. 4,228,158, U.S. 4,410,512 and U.S. 4,410,513.

Antibodies to the endogenous growth hormone release inhibitor, somatostatin (SRIF) have also been used to cause elevated GH levels. In this latter example, growth hormone levels are elevated by removing the endogenous GH-release inhibitor (SRIF) before it reaches the pituitary, where it inhibits the release of GH.

Each of these methods for promoting the elevation of growth hormone levels involve materials which are expensive to synthesize and/or isolate in sufficient purity for administration to a target animal. Short chain, low molecular weight, relatively simple polypeptides which are relatively inexpensive to prepare and have the ability to promote the release of growth hormone would be desirable because they should be readily and inexpensively prepared, easily modified chemically and/or physically, as well as readily purified and formulated; and they should have excellent transport properties.

### Objects of the Invention

It is, therefore, an object of the present invention to provide novel growth hormone releasing compounds which are capable of promoting the release and elevation of growth hormone levels in the blood of animals.

It is another object of the present invention to provide methods for promoting the release and/or elevation of growth hormone levels in the blood of animals.

These and other objects of the present invention will become apparent from inspection of the following description and claims.

### Statement of the Invention

In accordance with the present invention, we have discovered novel polypeptide compounds which promote the release of growth hormone in animals. The preparation, characterization and administration of these novel growth hormone releasing compounds will now be described in greater detail.

### Detailed Description of the Invention

The present invention is based on the discovery of short chain (i.e., six up to ten amino acid residues) polypeptides which promote the release and elevation of growth hormone levels in the blood of animals. The polypeptides contemplated to be within the scope of the present invention are defined by the following generic structure:

Ala-AA2-AA3-Trp-AA5-Y-Z,

wherein

AA2 is selected from the group consisting of DPhe, DTrp, 5-fluoro-D or D/LTrp; 6-fluoro-D or D/LTrp (i.e., wherein the indole ring is fluorinated at the 5- or 6-position), (formyl)DTrp (i.e., DTrp which is formylated at the indole nitrogen), *XTrp, wherein *XTrp is selected from the group consisting of the N-monomethylated DTrp isomers (i.e., ($N^{\alpha}$Me)DTrp and (indole NMe)DTrp), $D^{\alpha}$Nal and $D^{\beta}$Nal;

AA3 is selected from the group consisting of Ala, Gly and Ser;

AA5 is selected from the group consisting of DPhe and (NMe)DPhe;

Y is selected from the group consisting of:

(a) AA7, therein AA7 is selected from the group consisting of Arg, iLys, Lys and Orn; and

(b) -AA6-AA7, wherein AA6 is selected from the group consisting of all naturally occurring L-amino acids, dipeptides of the naturally occurring L-amino acids, e.g., Ala-Ala, and compounds of the formula:

$H_2N-(CH_2)_n-CO_2H$, wherein n = 1-12,

and wherein AA7 is as defined above; and

Z represents the C terminal end group of said polypeptide or the C terminal amino acid(s) plus end group, wherein Z is selected from the group consisting of $-CONH_2$, -COOH, -COOR, -CONHR, $-CONR_2$, $-CH_2OH$ and $-CH_2OR$, wherein R is an alkyl group having 1-6 carbon atoms or an aromatic ring having up to 12 carbon atoms; and wherein Z is alternatively selected from the group consisting of -Gly-Z', -Met-Z', -Lys-Z', -Cys-Z', -Gly-Tyr-Z', and -Ala-Tyr-Z', wherein Z' is selected from the group consisting of $-CONH_2$, -CONHR, -COOH, -COOR, $-CONR_2$, $-CH_2OH$, and $-CH_2OR$, wherein R is as defined above;

and organic or inorganic addition salts of any of said polypeptides;

wherein the amino acid residue abbreviations used are in accordance with the standard peptide nomenclature:

| | |
|---|---|
| Gly | = Glycine |
| Tyr | = L-Tyrosine |
| Ile | = L-Isoleucine |
| Glu | = L-Glutamic Acid |
| Thr | = L-Threonine |
| Phe | = L-Phenylalanine |
| Ala | = L-Alanine |
| Lys | = L-Lysine |
| Asp | = L-Aspartic Acid |
| Cys | = L-Cysteine |
| Arg | = L-Arginine |
| Gln | = L-Glutamine |
| Pro | = L-Proline |
| Leu | = L-Leucine |
| Met | = L-Methionine |
| Ser | = L-Serine |
| Asn | = L-Asparagine |
| His | = L-Histidine |
| Trp | = L-Tryptophan |
| Val | = L-Valine |
| DOPA | = 3,4-Dihydroxyphenylalanine |
| Met(O) | = Methionine Sulfoxide |
| Abu | = $\alpha$-Aminobutyric Acid |
| iLys | = $N^{\epsilon}$-Isopropyl-L-Lysine |
| 4-Abu | = 4-Aminobutyric Acid |
| Orn | = L-Ornithine |
| $D^{\alpha}$Nal | = $\alpha$-Naphthyl-D-Alanine |
| $D^{\beta}$Nal | = $\beta$-Naphthyl-D-Alanine |

All three letter amino acid abbreviations preceded by a "D" indicate the D-configuration of the amino acid residue, and abbreviations preceded by a "D/L" indicate a mixture of the D- and L-configurations of the designated amino acid. For purposes of this disclosure, glycine is considered to be included in the term "naturally occurring L-amino acids."

The flexibility associated with the choice of basic, neutral or acidic amino acid residues for amino acids AA2, AA3, AA5 and Y provides one with a great deal of control over the physiochemical properties of the desired peptide. Such flexibility provides important advantages for the formulation and delivery of the desired peptide to any given species. Additional flexibility can be imparted by the fact that the moieties R, Z and Z' can be varied as well, thereby providing added control over the physiochemical properties of the desired compound.

Preferred growth hormone releasing compounds employed in the practice of the present invention are selected from the group consisting of:

Ala-AA2-Ala-Trp-AA5-AA7-$NH_2$,

Ala-AA2-Ala-Trp-AA5-AA6-AA7-$NH_2$, and

organic or inorganic addition salts of any of said polypeptides; where AA2, AA5, AA6 and AA7 are as defined above.

These compounds are preferred because of their ease of synthesis, proven efficacy at promoting an increase in serum growth hormone levels, and their consequent appeal for commercial scale production and utilization. In addition, these compounds may be advantageous in having physiochemical properties which are desirable for the efficient delivery of such polypeptide compounds to a variety of animal species. Because of the flexibility made possible by the various substitutions at numerous positions of the invention polypeptide compounds, a wide range of delivery vehicles can be employed, by selecting the polar, neutral or non-polar nature of the C-terminal and center portions of these polypeptide compounds so as to be compatible with the desired method of delivery.

In a most preferred embodiment, the growth hormone releasing peptide employed in the practice of the present invention has the sequence:

Ala-AA2-Ala-Trp-AA5-AA7-$NH_2$; or

organic or inorganic addition salts thereof, where AA2, AA5 and AA7 are as defined above. A particularly preferred member of this most preferred group of compounds has the sequence:

Ala-DTrp-Ala-Trp-DPhe-Lys-$NH_2$, as well as organic or inorganic addition salts thereof.

These compounds are the presently most preferred because these shorter chain polypeptides are less expensive to synthesize, and these specific compounds have been shown to have a high level of potency at promoting the increase in serum growth hormone levels.

The compounds of this invention may be used to enhance blood GH levels in animals; enhance milk production in cows; enhance body growth in animals such as mammals (e.g., humans, sheep, bovines, and swine), as well as fish, fowl, other vertebrates and crustaceans; and increase wool and/or fur production in mammals. The amount of body growth is dependent upon the sex and age of the animal species, quantity and identity of the growth hormone releasing compound being administered, route of administration, and the like.

The novel polypeptide compounds of this invention can be synthesized according to the usual methods of solution and solid phase peptide chemistry, or by classical methods known in the art. The solid-phase synthesis is commenced from the C-terminal end of the peptide. A suitable starting material can be prepared, for instance, by attaching the required protected alpha-amino acid to a chloromethylated resin, a hydroxymethyl resin, a benzhydrylamine (BHA) resin, or a para-methyl-benzylhydrylamine (p-Me-BHA) resin. One such chloromethyl resin is sold under the tradename BIOBEADS SX-1 by Bio Rad Laboratories, Richmond, Calif. The preparation of the hydroxymethyl resin is described by Bodansky et al., Chem. Ind. - (London) 38, 1597 (1966). The BHA resin has been described by Pietta and Marshall, Chem. Comm., 650 (1970) and is commercially available from Peninsula Laboratories, Inc., Belmont, California.

After the initial attachment, the alpha-amino protecting group can be removed by a choice of acidic reagents, including trifluoroacetic acid (TFA) or hydrochloric acid (HCl) solutions in organic solvents at room temperature. After removal of the alpha-amino protecting group, the remaining protected amino acids can be coupled stepwise in the desired order. Each protected amino acid can be generally reacted in about a 3-fold excess using an appropriate carboxyl group activator such as dicyclohexylcarbodiimide (DCC) or diisopropyl carbodiimide (DIC) in solution, for example, in methylene chloride ($CH_2Cl_2$) or dimethylformamide (DMF) and mixtures thereof.

After the desired amino acid sequence has been completed, the desired peptide can be cleaved from the resin support by treatment with a reagent such as hydrogen fluoride (HF) which not only cleaves the peptide from the resin, but also cleaves most commonly used side-chain protecting groups. When a chloromethyl resin or hydroxymethyl resin is used, HF treatment results in the formation of the free peptide acid. When the BHA or p-Me-BHA resin is used, HF treatment results directly in free peptide amides.

The solid-phase procedure discussed above is well known in the art and has been described by Stewart and Young, Solid Phase Peptide Synthesis: Second Edn. (Pierce Chemical Co., Rockford, IL, 1984).

Some of the well known solution methods which can be employed to synthesize the peptide moieties of the instant invention are set forth in Bodansky et al., Peptide Synthesis, 2nd Edition, John Wiley & Sons, New York, N.Y. 1976.

In accordance with another embodiment of the present invention, a method is provided for promoting release and/or elevation of growth hormone levels in the blood of an animal. Said method comprises administering to an animal an effective dose of at least one of the above-described polypeptides.

The compounds of this invention can be administered by oral, parenteral (intramuscular (i.m.), intraperitoneal (i.p.), intravenous (i.v.) or subcutaneous (s.c.) injection), nasal, vaginal, rectal or sublingual

routes of administration and can be formulated in dose forms appropriate for each route of administration.

Solid dose forms for oral administration include capsules, tablets, pills, powders and granules. In such solid dose forms, the active compound is mixed with at least one inert carrier such as sucrose, lactose, or starch. Such dose forms can also comprise, as is normal practice, additional substances other than inert diluents, e.g., lubricating agents such as magnesium stearate. In the case of capsules, tablets and pills, the dose forms may also comprise buffering agents. Tablets and pills can additionally be prepared with enteric coatings.

Liquid dose forms for oral administration include emulsions, solutions, suspensions, syrups, the elixirs containing inert diluents commonly used in the art, such as water. Besides, such inert diluents, compositions can also include adjuvants, such as wetting agents, emulsifying and suspending agents, and sweetening, flavoring, and perfuming agents.

Preparations according to this invention for parenteral administration include sterile aqueous or non-aqueous solutions, suspensions, or emulsions. Examples of non-aqueous solvents or vehicles are propylene glycol, polyethylene glycol, vegetable oils, such as olive oil and corn oil, gelatin, and injectable organic esters such as ethyl oleate. Such dose forms may also contain adjuvants such as preserving, wetting, emulsifying, and dispersing agents. They may be sterilized by, for example, filtration through a bacteria-retaining filter, by incorporating sterilizing agents into the compositions, by irradiating the compositions, or by heating the compositions. They can also be manufactured in a medium of sterile water, or some other sterile injectable medium immediately before use.

As suggested in our copending applications Serial No. 861,968 and S.N. 37,275, incorporated by reference herein, the novel compounds of the present invention are also believed to be useful when administered in combination with growth hormone releasing hormone (i.e., naturally occurring growth hormone releasing hormone, analogs and functional equivalents thereof), as well as in combination with other compounds which promote the release of growth hormone. Such combinations represent an especially preferred means to administer the growth hormone releasing peptides of the present invention because the combination promotes the release of much more growth hormone than is predicted by the summation of the individual responses for each component of the combination, i.e., the combination provides a synergistic response relative to the individual component.

Combinations effective to cause the release and elevation of the level of growth hormone in the blood of an animal comprise an effective amount of polypeptides selected from group 2 polypeptides and at least one of Group 1 polypeptides or Group 3 polypeptides,

wherein Group 1 polypeptides are selected from any of the naturally occurring growth hormone releasing hormones and functional equivalents thereof, wherein said polypeptides act at the growth hormone releasing hormone receptor of mammals and other vertebrates, and crustaceans;

Group 2 polypeptides are selected from any of the polypeptides having the structure:

Ala-AA2-AA3-Trp-AA5-Y-Z,

wherein AA2 is selected from the group consisting of DPhe, DTrp, 5-fluoro-D or D/LTrp; 6-fluoro-D or D/LTrp (i.e., wherein the indole ring is fluorinated at the 5- or 6-position), (formyl)DTrp (i.e., DTrp which is formylated at the indole nitrogen), *XTrp, wherein *XTrp is selected from the group consisting of the N-monomethylated DTrp isomers (i.e., ($N^{\alpha}$Me)DTrp and (indole NMe)DTrp), $D^{\alpha}$Nal and $D^{\beta}$Nal;

AA3 is selected from the group consisting of Ala, Gly and Ser;

AA5 is selected from the group consisting of DPhe and (NMe)DPhe;

Y is selected from the group consisting of:

(a) AA7, wherein AA7 is selected from the group consisting of Arg, iLys, Lys and Orn; and

(b) -AA6-AA7, wherein AA6 is selected from the group consisting of all naturally occurring L-amino acids, dipeptides of the naturally occurring L-amino acids, e.g., Ala-Ala, and compounds of the formula:

$H_2N-(CH_2)_n-CO_2H$,

wherein n = 1-12

and wherein AA7 is as defined above; and

Z represents the C terminal end group of said polypeptide or the C terminal amino acid(s) plus end group, wherein Z is selected from the group consisting of $-CONH_2$, -COOH, -COOR, -CONHR, $-CONR_2$, $-CH_2OH$ and $-CH_2OR$, wherein R is an alkyl group having 1-6 carbon atoms or an aromatic ring having up to 12 carbon atoms; and wherein Z is alternatively selected from the group consisting of -Gly-Z', -Met-Z', -Lys-Z', -Cys-Z', -Gly-Tyr-Z', and -Ala-Tyr-Z', wherein Z' is selected from the group consisting of $-CONH_2$, -COOH, -CONHR, -COOR, $-CONR_2$, $-CH_2OH$, and $-CH_2OR$, wherein R is as defined above;

and organic or inorganic addition salts of any of said polypeptides;

wherein the amino acid residue abbreviations used are in accordance with the standard peptide nomenclature:

| | | |
|---|---|---|
| Gly | = | Glycine |
| Tyr | = | L-Tyrosine |
| Ile | = | L-Isoleucine |
| Glu | = | L-Glutamic Acid |
| Thr | = | L-Threonine |
| Phe | = | L-Phenylalanine |
| Ala | = | L-Alanine |
| Lys | = | L-Lysine |
| Asp | = | L-Aspartic Acid |
| Cys | = | L-Cysteine |
| Arg | = | L-Arginine |
| Gln | = | L-Glutamine |
| Pro | = | L-Proline |
| Leu | = | L-Leucine |
| Met | = | L-Methionine |
| Ser | = | L-Serine |
| Asn | = | L-Asparagine |
| His | = | L-Histidine |
| Trp | = | L-Tryptophan |
| Val | = | L-Valine |
| Abu | = | $\alpha$-Aminobutyric Acid |
| Sar | = | Sarcosine |
| Sar-ol | = | Sarcosine Alcohol |
| DOPA | = | 3,4-Dihydroxyphenylalanine |
| Gly-ol | = | 2-Aminoethanol |
| Hyp | = | trans-4-Hydroxy-L-Proline |
| Met(O) | = | Methionine sulfoxide |
| Met(O)-ol | = | Methionine sulfoxide alcohol |
| Thz | = | L-Thiazolidine-4-carboxylic Acid |
| iLys | = | $N^{\epsilon}$-Isopropyl-L-Lysine |
| 4-Abu | = | 4-Aminobutyric Acid |
| Orn | = | L-Ornithine |
| $D^{\alpha}$Nal | = | $\alpha$-Naphthyl-D-Alanine |
| $D^{\beta}$Nal | = | $\beta$-Naphthyl-D-Alanine |

All three letter amino acid abbreviations preceded by a "D" indicate the D-configuration of the amino acid residue; abbreviations preceded by a "D/L" indicate a mixture of the D- and L-configurations of the designated amino acids; and glycine is included in the scope of the term "naturally occurring L-amino acids";

Group 3 polypeptides are selected from any of the polypeptides having the structure:

Tyr-DArg-Phe-$NH_2$
Tyr-DAla-Phe-$NH_2$;
Tyr-DArg($NO_2$)-Phe-$NH_2$;
Tyr-DMet(O)-Phe-$NH_2$;
Tyr-DAla-Phe-Gly-$NH_2$;
Tyr-DArg-Phe-Gly-$NH_2$;
Tyr-DThr-Phe-Gly-$NH_2$;
Phe-DArg-Phe-Gly-$NH_2$;
Tyr-DArg-Phe-Sar;
Tyr-DAla-Gly-Phe-$NH_2$;
Tyr-DArg-Gly-Trp-$NH_2$;
Tyr-DArg($NO_2$)-Phe-Gly-$NH_2$;
Tyr-DMet(O)-Phe-Gly-$NH_2$;
(NMe)Tyr-DArg-Phe-Sar-$NH_2$;
Tyr-DArg-Phe-Gly-ol;
Tyr-DArg-Gly-(NMe)Phe-$NH_2$;
Tyr-DArg-Phe-Sar-ol

Tyr-DAla-Phe-Sar-ol
Tyr-DAla-Phe-Gly-Tyr-$NH_2$;
Tyr-DAla-(NMe)Phe-Gly-Met(O)-ol;
Tyr-DArg-(NMe)Phe-Gly-Met(O)-ol;
Gly-Tyr-DArg-Phe-Gly-$NH_2$;
Tyr-DThr-Gly-Phe-Thz-$NH_2$;
Gly-Tyr-DAla-Phe-Gly-$NH_2$;
Tyr-DAla-Phe-Gly-ol;
Tyr-DAla-Gly-(NMe)Phe-Gly-ol;
Tyr-DArg-Phe-Sar-$NH_2$;
Tyr-DAla-Phe-Sar-$NH_2$;
Tyr-DAla-Phe-Sar;
Tyr-DAla-Gly-(NMe)Phe-$NH_2$;
Sar-Tyr-DArg-Phe-Sar-$NH_2$;
Tyr-DCys-Phe-Gly-DCys-$NH_2$ (cylic disulfide);
Tyr-DCys-Phe-Gly-DCys-$NH_2$ (free dithiol);
Tyr-DCys-Gly-Phe-DCys-$NH_2$ (cyclic disulfide);
Tyr-DCys-Gly-Phe-DCys-$NH_2$ (free dithiol);
Tyr-DAla-Phe-Gly-Tyr-Pro-Ser-$NH_2$;
Tyr-DAla-Phe-Sar-Tyr-Pro-Ser-$NH_2$;
Tyr-DAla-Phe-Sar-Phe-Pro-Ser-$NH_2$;
Tyr-DAla-Phe-Gly-Tyr-Hyp-Ser-$NH_2$;
Tyr-DAla-Phe-Sar-Tyr-Hyp-Ser-$NH_2$;
Tyr-DAla-Phe-Sar-Phe-Hyp-Ser-$NH_2$;
Tyr-DArg-Phe-Gly-Tyr-Hyp-Ser-$NH_2$;
Tyr-DArg-Phe-Sar-Tyr-Pro-Ser-$NH_2$;
Tyr-DArg-Phe-Sar-Tyr-Hyp-Ser-$NH_2$;
Tyr-DArg-Phe-Gly-Tyr-Pro-Ser-$NH_2$; and
organic or inorganic addition salts of any of said polypeptides of Group 3;

wherein said combination is administered in a ratio such that said combination is effective to cause the synergistic release and elevation of growth hormone in the blood of such animal.

Preferred Group 1 polypeptides are selected from any of the polypeptides:

(a) having the following amino acid sequences in Positions 1-44 (numbered from N terminus to C terminus):

(#144) YADAIFTNSYRKVLGQLSARKLLQDIMSRQQGE-SNQERGARARL-X,
(#145) YADAIFTNSYRKVLGQLSARKLLQDIMSRQQGE-RNQEQGARVRL-X,
(#146) YADAIFTNSYRKVLGQLSARKLLQDIMNRQQGE-RNQEQGAKVRL-X,
(#148) YADAIFTNSYRKILGQLSARKLLQDIMNRQQGE-RNQEQGAKVRL-X,
(#149) HADAIFTSSYRRILGQLYARKLLHEIMNRQQGE-RNQEQRSRFN-X;

and functional equivalents thereof:
wherein the C-terminal amino acid has the following truncated general formula

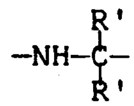

wherein each R' independently represents the substituents of the particular amino acid residue, e.g.: hydrogen, alkyl, aryl, amino or acid substituents; X denotes the C terminal end group and is selected from $-CONH_2$, -COOH, -COOR, -CONRR, $-CH_2OH$, and $-CH_2OR$, where R is an alkyl group having 1 to 6 carbon atoms or an aromatic ring having up to 12 carbon atoms; and wherein the amino acid residue abbreviations used are in accordance with the standard peptide nomenclature:

G = Gly (Glycine),
Y = Tyr (L-Tyrosine),
I = Ile (L-Isoleucine),
E = Glu (L-Glutamic Acid),
T = Thr (L-Threonine),
F = Phe (L-Phenylalanine),
A = Ala (L-Alanine),
K = Lys (L-Lysine),
D = Asp (L-Aspartic Acid),
C = Cys (L-Cysteine),
R = Arg (L-Arginine),
Q = Gln (L-Glutamine),
P = Pro (L-Proline),
L = Leu (L-Leucine),
M = Met (L-Methionine),
S = Ser (L-Serine),
N = Asn (L-Asparagine),
H = His (L-Histidine),
W = Trp (L-Tryptophan), and
V = Val (L-Valine);
Nle = Norleucine
Sar = Sarcosine
Sar-ol = Sarcosine Alcohol
Gly-ol = 2-Aminoethanol
Met(O) = Methionine Sulfoxide

(b) any one of said (a) polypeptides having the following amino acid substitutions:
Position 1 of (#144-#148) is DTyr or His;
Position 1 of (#149) is Tyr or DHis;
Position 2 of (#144-#149) is (NMe)DAla or Aib or DAla;
Position 3 of (#144-#149) is DAsp;
Position 4 of (#144-#149) is DAla; and
Position 1 + 2 of (#144-#149) is;
$DTyr^1 + DAla^2$, $DTyr^1 + (NMe)DAla^2$, or $DTyr^1 + Aib^2$;
(c) any one of said (a) or (b) polypeptides having a substitution of Nle for Met at Position 27;
(d) any one of said (a), (b) or (c) polypeptides in which the N-terminus $-NH_2$ is replaced by -NHCOR and wherein R is an alkyl group having 1 to 6 carbon atoms, or an aromatic ring having up to 12 carbon atoms;
(e) fragments of any one of said (a), (b), (c) or (d) polypeptides which contain at least the amino acid residues of Positions 1-29;
(f) having the following specific amino acid sequences in Positions 1-29 (numbered from N terminus to C terminus):

YADAIFTNSYRKVLQQLAARKLLQDIMSR-X,
YADAIFTNSYRKVLQQLLARKLLQDIMSR-X,
YSDAIFSNAYRKILQQLLARKLLQDIMQR-X,
YADAIFSNAYRKILQQLLARKLLQDIMQR-X,
YADAIFSSAYRRLLAQLASRRLLQELLAR-X,
YADAIFTNCYRKVLCQLSARKLLQDIMSR-X (linear dithiol), and
YADAIFTNCYRKVLCQLSARKLLQDIMSR-X (cyclic disulfide);

wherein the C-terminal amino acid and X are as defined above; and modification of any one of these group (f) compounds in accordance with the modifications set forth in (b), (c) and (d) above; and

(g) organic or inorganic addition salts of any of said (a), (b), (c), (d), (e) or (f) polypeptides of Group 1.

For further detail on the administration of combinations of growth hormone releasing peptides, those of skill in the art are referred to the above-cited applications.

The amount of polypeptide or combination of polypeptides of the present invention administered will vary depending on numerous factors, e.g., the particular animal treated, its age and sex, the desired therapeutic affect, the route of administration and which polypeptide or combination of polypeptides are employed. In all instances, however, a dose effective to promote release and elevation of growth hormone level in the blood of the recipient animal is used. Ordinarily, this dose level falls in the range of between about 0.1 µg up to 10 mg of total polypeptide per kg of body weight. In general, the administration of combinations of growth hormone releasing peptides will allow for lower doses of the individual growth hormone releasing compounds to be employed relative to the dose levels required for individual growth hormone releasing compounds in order to obtain a similar response, due to the synergistic effect of the combination.

Also included within the scope of the present invention are compositions comprising, as an active ingredient, the organic and inorganic addition salts of the above described polypeptides and combinations thereof; optionally, in association with a carrier, diluent, slow release matrix, or coating.

The organic or inorganic addition salts of the growth hormone releasing compounds and combinations thereof contemplated to be within the scope of the present invention include salts of such organic moieties as acetate, trifluoroacetate, oxalate, valerate, oleate, laurate, benzoate, lactate, tosylate, citrate, maleate, fumarate, succinate, tartrate, naphthalate, and the like; and such inorganic moieties as Group I (i.e., alkali metal salts), Group II (i.e., alkaline earth metal salts), ammonium and protamine salts, zinc, iron, and the like with counterions such as the chloride, bromide, sulfate, phosphate and the like, as well as the organic moieties referred to above.

Pharmaceutically acceptable salts are preferred when administration to human subjects is contemplated. Such salts include the non-toxic alkali metal, alkaline earth metal and ammonium salts commonly used in the pharmaceutical industry including the sodium, potassium, lithium, calcium, magnesium, barium, ammonium and protamine salts which are prepared by methods well known in the art. The term also includes non-toxic acid addition salts which are generally prepared by reacting the compounds of this invention with a suitable organic or inorganic acid. Representative salts include the hydrochloride, hydrobromide, sulfate, bisulfate, acetate, oxalate, valerate, oleate, laurate, borate, benzoate, lactate, phosphate, tosylate, citrate, maleate, fumarate, succinate, tartrate, napsylate, and the like.

The invention will now be described in greater detail by reference to the following non-limiting examples.

EXAMPLE 1 - Synthesis of the Growth Hormone Releasing Peptides

Paramethyl-benzhydrylamine hydrochloride (pMe-BHA•HCl) resin is placed in a reaction vessel on a commercially available automated peptide synthesizer. The resin is substituted with free amine up to a loading of about 5 mmoles per gram. The compounds are prepared by coupling individual amino acids starting at the carboxy terminus of the peptide sequence using an appropriate activing agent, such as N,N'-dicyclohexylcarbodiimide (DCC). The alpha amine functionalities of individual amino acids are protected, for example, as the t-butyloxycarbonyl derivative (t-Boc) and the reactive side chain functionalities can be protected as outlined in Table 1.

Table 1

| Side Chain Protecting Groups Suitable for Solid Phase Peptide Synthesis | |
|---|---|
| Arginine: | $N^g$-Tosyl |
| Aspartic Acid: | O-Benzyl |
| Cysteine: | S-para-Methylbenzyl |
| Glutamic Acid: | O-Benzyl |
| Histidine: | $N^{im}$-Tosyl |
| Lysine: | $N^\epsilon$-2,4-Dichlorobenzyloxycarbonyl |
| Methionine: | S-Sulfoxide |
| Serine: | O-Benzyl |
| Threonine: | O-Benzyl |
| Tryptophan: | $N^{in}$-Formyl |
| Tyrosine: | 0-2,6-Dichlorobenzyl |

Prior to incorporation of the initial amino acid, the resin is agitated three times (about one minute each) with dichloromethane ($CH_2Cl_2$; about 10 mL/gm of resin), neutralized with three agitations (about two minutes each) of N,N-diisopropylethylamine (DIEA) in dichloromethane (10:90; about 10 mL/gm of resin) and agitated three times (about one minute each) with dichloromethane (about 10 mL/gm of resin). The initial and each of the subsequent amino acids are coupled to the resin using a preformed symmetrical anhydride using about 3.0 times the total amount of the binding capacity of the resin of a suitably protected amino acid and about 1.5 times the total amount of the binding capacity of the resin of DCC in an appropriate amount of dichloromethane. For amino acids with a low dichloromethane solubility, N,N-dimethylformamide (DMF) is added to achieve a homogenous solution. Generally, the symmetrical anhydride is prepared up to 30 minutes prior to introduction into the reaction vessel at room temperature or below. The dicyclohexylurea that forms upon preparation of the symmetrical anhydride is removed via gravity filtration of the solution into the reaction vessel. Progress of the coupling of the amino acid to the resin is commonly monitored via a color test using a reagent such as ninhydrin (which reacts with primary and secondary amines. Upon complete coupling of the protected amino acid to the resin (>99%), the alpha amine protecting group is removed by treatment with acidic reagent(s). A commonly used reagent consists of a solution of trifluoroacetic acid (TFA), and anisole in dichloromethane (45:2:53).

After the desired amino acid sequence has been completed, the desired peptide can be cleaved from the resin support by treatment with a reagent such as hydrogen fluoride (HF) which not only cleaves the peptide from the resin, but also cleaves most commonly used side-chain protecting groups. When a chloromethyl resin or hydroxymethyl resin is used, HF treatment results in the formation of the free peptide acid. When the BHA or p-Me-BHA resin is used, HF treatment results directly in free peptide amides.

The complete procedure for incorporation of each individual amino acid residue onto the resin is outlined in Table 2.

TABLE 2

| Procedure for Incorporation of Individual Amino Acids onto a Resin | | | |
|---|---|---|---|
| | Reagent | Agitations | Time/Agitation |
| 1. | Dichloromethane | 3 | 1 min. |
| 2. | TFA, Anisole, Dichloromethane (45:2:53) | 1 | 2 min. |
| 3. | TFA, Anisole, Dichloromethane (45:2:53) | 1 | 20 min. |
| 4. | Dichloromethane | 3 | 1 min. |
| 5. | DIEA, Dichloromethane (10:90) | 3 | 2 min. |
| 6. | Dichloromethane | 3 | 1 min. |
| 7. | Preformed symmetrical anhydride | 1 | 15-120 min.* |
| 8. | Dichloromethane | 3 | 1 min. |
| 9. | iso-Propanol | 3 | 1 min. |
| 10. | Dichloromethane | 3 | 1 min. |
| 11. | Monitor progress of the coupling reaction** | | |
| 12. | Repeat Steps 1-12 for each individual amino acid | | |

*Coupling time depends upon the individual amino acid.
**The extent of coupling can be generally monitored by a color test. If the coupling is incomplete, the same amino acid can be recoupled by repeating Steps 7-11. If the coupling is complete the next amino acid can be coupled.

EXAMPLE 2 - In Vivo GH Release in Rats

Immature female Sprague-Pawley rats were obtained from the Charles River Laboratories (Wilmington, MA). After arrival they were housed at 25° C with a 14:10 hr light:dark cycle. Water and Purina rat chow were available ad libitum. Pups were kept with their mothers until 21 days of age.

Twenty-six day old rats, six rats per treatment group, were anesthetized interperitoneally with 50 mg/kg of pentobarbital 20 minute prior to i.v. treatment with peptide. Normal saline with 0.1% gelatin was the vehicle for intravenous (i.v.) injections of the peptides. The anesthetized rats, weighing 55-65 grams, were injected i.v. with the quantity of growth hormone releasing compounds indicated in Table 3. Injection was made as a 0.1 mL solution into the jugular vein.

All animals were sacrificed by guillotine 10 minutes after the final test injection (see Table 3). Trunk blood for the determination of blood GH levels was collected following decapitation. After allowing the blood to clot, it was centrifuged and the serum was separated from the clot. Serum was kept frozen until the day of sampling for radioimmunoassay (RIA) determination of growth hormone levels according to the following procedure, as developed by the National Institute of Arthritis, Diabetes and Digestive and Kidney Diseases (NIADDK).

Reagents are generally added to the RIA analysis tubes at a single sitting, at refrigerator temperature (about 4°C) in the following sequence:

(a) buffer,
(b) "cold" (i.e., non-radioactive) standard or unknown serum sample to be analyzed,
(c) radio-iodinated growth hormone antigen, and
(d) growth hormone antiserum.

Reagent addition is generally carried out so that there is achieved a final RIA tube dilution of about 1:30,000 (antiserum to total liquid volume; vol:vol).

The mixed reagents are then typically incubated at room temperature (about 25°C) for about 24 hours prior to addition of a second antibody (e.g., goat or rabbit anti-monkey gamma globulin serum) which binds to and causes precipitation of the complexed growth hormone antiserum. Precipitated contents of the RIA tubes are then analyzed for the number of counts in a specified period of time in a gamma scintillation counter. A standard curve is prepared by plotting number of radioactive counts versus growth hormone (GH) level. GH levels of unknowns are then determined by reference to the standard curve.

Serum GH was measured by RIA with reagents provided by the National Hormone and Pituitary Program.

Serum levels in Table 3 are recorded in ng/mL in terms of the rat GH standard of 0.61 International Units/mg (IU/mg). Data is recorded as the mean +/-standard error of the mean (SEM). Statistical analysis

was performed with Student's t-test. In Table 3 the results shown are the average of studies with six rats.

Table 3

| In Vivo GH Release (ng/mL) Promoted by Growth Hormone Releasing Compounds in Pentobarbital Anesthetized Rats (Animals Sacrificed 10 Minutes After Final Injection) | | | |
|---|---|---|---|
| Column A Growth Hormone Releasing Compounds | Total Dose (μg) | Control GH ng/mL | GH Released by Compound in Column A ng/mL |
| His-DTrp-Ala-Trp-DPhe--Lys-$NH_2$* | 0.3 | 208±47 | 660±128 |
| | 1.0 | 208±47 | 1669±257 |
| | 3.0 | 208±47 | 3078±629 |
| | 0.3 | 142±43 | 533± 77 |
| | 1.0 | 142±43 | 1656±213 |
| | 3.0 | 142±43 | 2653±378 |
| | 0.3** | 108±17 | 764± 62 |
| | 1.0** | 108±17 | 2614±349 |
| | 3.0** | 108±17 | 3215±547 |
| His-Ala-DTrp-Ala-Trp-D-Phe-Lys-$NH_2$* | 0.3 | 151±16 | 261± 32 |
| | 1.0 | 151±16 | 830±103 |
| | 3.0 | 151±16 | 2365±478 |
| | 3.0 | 111±25 | 2588±341 |
| Ala-DTrp-Ala-Trp-DPhe--Lys-$NH_2$ | 0.3 | 208±47 | 265± 96 |
| | 1.0 | 208±47 | 754±164 |
| | 3.0 | 208±47 | 1449±238 |
| | 0.3 | 142±43 | 303± 41 |
| | 1.0 | 142±43 | 729±115 |
| | 3.0 | 142±43 | 1164±215 |
| | 10.0 | 142±43 | 2353±149 |
| | 30.0 | 142±43 | 2484±110 |
| | 1.0** | 108±17 | 745± 68 |
| | 3.0** | 108±17 | 1673±352 |
| | 10.0** | 108±17 | 2388±269 |

*Comparison Peptides
**Doses so noted were administered to 29 day old female rats.

In Table 3, a compound of the invention is shown to promote the release and elevation of growth hormone levels in the blood of rats to which such compounds have been administered. The surprising growth hormone releasing activity of this compound is potentially quite valuable since a short-chain, low molecular weight polypeptide with the relatively stable and inexpensive amino acid alanine at the amino-terminus should prove to be a low cost means to enhance growth hormone levels in animals.

EXAMPLE 3 - Administration of a Combination of GH-Releasing Compounds

The procedure of Example 2 was repeated, except the rats were not anesthetized nor were they pretreated with pentobarbital, and a combination of peptides were administered to the rats. The compounds administered, the dose levels employed and results are set forth in Table 4.

TABLE 4

In Vivo Synergistic Effects in Unanesthetized Rats of Invention Compound with Group 1* Compound

| Compound Administered; Dose (μg)* | GH Released, mg/mL |
|---|---|
| Control | 9±0.9 |
| Invention Compound, 10 | 75± 21 |
| 30 | 355±141 |
| Comparison Compound, 10 | 256± 66 |
| Group 1 Compound, 10 | 144± 19 |

TABLE 4 (Cont'd.)

In Vivo Synergistic Effects in Unanesthetized Rats of Invention Compound with Group 1* Compound

| Compound Administered; Dose (μg)* | GH Released, mg/mL |
|---|---|
| Invention + Group 1, 10+10 | 2566±334 |
| 30+10 | 2020±214 |
| Comparison + Group 1, 10+10 | 2488±285 |

*Group 1 compounds are described in detail in S.N. 861,968 and 37,275, which have been incorporated by reference herein. All compounds employed for this example have the following sequences:

Invention Compound:

Ala–DTrp–Ala–Trp–DPhe–Lys–$NH_2$

Comparison Compound:

His–DTrp–Ala–Trp–DPhe–Lys–$NH_2$

Group 1 Compound:

Tyr–Ala–Asp–Ala–Ile–Phe–Thr–Asn–Ser–Tyr–Arg–Lys–Val–Leu–Gly–Gln–Leu–Ser–Ala–Arg–Lys–Leu–Leu–Gln–Asp–Ile–Nle–Ser–Arg–$NH_2$

---

The results in Table 4 demonstrate that invention compound displays a similar synergistic response to that obtained with comparison compound (which has previously been shown to give a synergistic response) when administered in combination with an exemplary Group 1 compound.

The invention has been described in detail with particular reference to preferred embodiments thereof, but it will be understood that variations and modifications can be effected within the spirit and scope of the invention.

## Claims

**1.** A polypeptide capable of promoting the release and elevation of growth hormone levels in the blood of a recipient animal, wherein said polypeptide is selected from the group consisting of polypeptides defined by the generic structure:

Ala-AA2-AA3-Trp-AA5-Y-Z,

wherein AA2 is selected from the group consisting of DPhe, DTrp, 5-fluoro-D or D/LTrp; 6-fluoro-D or D/LTrp (i.e., wherein the indole ring is fluorinated at the 5- or 6-position), (formyl)DTrp (i.e., DTrp which is formylated at the indole nitrogen), *XTrp, wherein *XTrp is selected from the group consisting of the N-monomethylated DTrp isomers (i.e., ($N^{\alpha}$Me)DTrp and (indole NMe)DTrp), $D^{\alpha}$Nal and $D^{\beta}$Nal;
AA3 is selected from the group consisting of Ala, Gly and Ser;
AA5 is selected from the group consisting of DPhe and (NMe)DPhe;
Y is selected from the group consisting of:

(a) AA7, wherein AA7 is selected from the group consisting of Arg, iLys, Lys and Orn; and
(b) -AA6-AA7, wherein AA6 is selected from the group consisting of all naturally occurring L-amino acids, dipeptides of the naturally occurring L-amino acids, e.g., Ala-Ala, and compounds of the formula:

$H_2N-(CH_2)_n-CO_2H$,

wherein n = 1-12,
and wherein AA7 is as defined above; and

Z represents the C terminal end group of said polypeptide or the C terminal amino acid(s) plus end group, wherein Z is selected from the group consisting of $-CONH_2$, -COOH, -COOR, -CONHR, $-CONR_2$, $-CH_2OH$ and $-CH_2OR$, wherein R is an alkyl group having 1-6 carbon atoms or an aromatic ring having up to 12 carbon atoms; and wherein Z is alternatively selected from the group consisting of -Gly-Z', -Met-Z', -Lys-Z', -Cys-Z', -Gly-Tyr-Z', and -Ala-Tyr-Z', wherein Z' is selected from the group consisting of $-CONH_2$, -COOH, -CONHR, -COOR, $-CONR_2$, $-CH_2OH$, and $-CH_2OR$, wherein R is as defined above; and organic or inorganic addition salts of any of said polypeptides;
wherein the amino acid residue abbreviations used are in accordance with the standard peptide nomenclature:

| | | |
|---|---|---|
| Gly | = | Glycine |
| Tyr | = | L-Tyrosine |
| Ile | = | L-Isoleucine |
| Glu | = | L-Glutamic Acid |
| Thr | = | L-Threonine |
| Phe | = | L-Phenylalanine |
| Ala | = | L-Alanine |
| Lys | = | L-Lysine |
| Asp | = | L-Aspartic Acid |
| Cys | = | L-Cysteine |
| Arg | = | L-Arginine |
| Gln | = | L-Glutamine |
| Pro | = | L-Proline |
| Leu | = | L-Leucine |
| Met | = | L-Methionine |
| Ser | = | L-Serine |
| Asn | = | L-Asparagine |
| His | = | L-Histidine |
| Trp | = | L-Tryptophan |
| Val | = | L-Valine |
| DOPA | = | 3,4-Dihydroxyphenylalanine |
| Met(O) | = | Methionine Sulfoxide |
| Abu | = | $\alpha$-Aminobutyric Acid |

iLys = $N^{\epsilon}$-Isopropyl-L-lysine
4-Abu = 4-Aminobutyric acid
Orn = L-Ornithine
$D^{\alpha}$Nal = $\alpha$-naphthyl-D-alanine
$D^{\beta}$Nal = $\beta$-naphthyl-D-alanine

All three letter amino acid abbreviations preceded by a "D" indicate the D-configuration of the amino acid residue; abbreviations preceded by a "D/L" indicate a mixture of the D- and L-configurations of the designated amino acids; and glycine is included in the scope of the term "naturally occurring L-amino acids".

**2.** A polypeptide in accordance with Claim 1, wherein said polypeptide is selected from the group consisting of:
Ala-AA2-Ala-Trp-AA5-AA7-$NH_2$,
Ala-AA2-Ala-Trp-AA5-AA6-AA7-$NH_2$,
and
organic or inorganic addition salts of any of said polypeptides; wherein AA2, AA5, AA6 and AA7 are as defined above.

**3.** A polypeptide in accordance with Claim 1 wherein said polypeptide has the sequence:
Ala-AA2-Ala-Trp-AA5-AA7-$NH_2$, or
organic or inorganic addition salts thereof, wherein AA2, AA5 and AA7 are as defined above.

**4.** A polypeptide in accordance with Claim 1 wherein said polypeptide has the sequence:
Ala-DTrp-Ala-Trp-DPhe-Lys-$NH_2$; or
organic or inorganic addition salts thereof.

**5.** Method of promoting the release and elevation of blood growth hormone levels in animals by administering thereto an effective amount of at least one of the polypeptides set forth in Claim 1.

**6.** Method of promoting the release and elevation of blood growth hormone levels in animals by administering thereto an effective amount of at least one of the polypeptides set forth in Claim 2.

**7.** Method of promoting the release and elevation of blood growth hormone levels in animals by administering thereto an effective amount of at least one of the polypeptides set forth in Claim 3.

**8.** Method of promoting the release and elevation of blood growth hormone levels in animals by administering thereto an effective amount of at least one of the polypeptides set forth in Claim 4.

**9.** A combination effective to cause the release and elevation of the level of growth hormone in the blood of an animal, the combination comprising an effective amount of polypeptides selected from Group 2 polypeptides and at least one of Group 1 polypeptides or Group 3 polypeptides,
wherein Group 1 polypeptides are selected from any of the naturally occurring growth hormone releasing hormones and functional equivalents thereof, wherein said polypeptides act at the growth hormone releasing hormone receptor of mammals and other vertebrates, and crustaceans;
Group 2 polypeptides are selected from any of the polypeptides having the structure:
Ala-AA2-AA3-Trp-AA5-Y-Z,
wherein AA2 is selected from the group consisting of DPhe, DTrp, 5-fluoro-D or D/LTrp; 6-fluoro-D or D/LTrp (i.e., wherein the indole ring is fluorinated at the 5- or 6-position), (formyl)DTrp (i.e., DTrp which is formylated at the indole nitrogen), *XTrp, wherein *XTrp is selected from the group consisting of the N-monomethylated DTrp isomers (i.e., ($N^{\alpha}$Me)DTrp and (indole NMe)DTrp), $D^{\alpha}$Nal and $D^{\beta}$Nal;
AA3 is selected from the group consisting of Ala, Gly and Ser;
AA5 is selected from the group consisting of DPhe and (NMe)DPhe;
Y is selected from the group consisting of:
(a) AA7, wherein AA7 is selected from the group consisting of Arg, iLys, Lys and Orn; and
(b) -AA6-AA7, wherein AA6 is selected from the group consisting of all naturally occurring L-amino acids, dipeptides of the naturally occurring L-amino acids, e.g., Ala-Ala, and compounds of the formula:

$H_2N-(CH_2)_n-CO_2H$,

wherein n = 1-12,
and wherein AA7 is as defined above; and

Z represents the C terminal end group of said polypeptide or the C terminal amino acid(s) plus end group, wherein Z is selected from the group consisting of $-CONH_2$, -COOH, -COOR, -CONHR, $-CONR_2$, $-CH_2OH$ and $-CH_2OR$, wherein R is an alkyl group having 1-6 carbon atoms or an aromatic ring having up to 12 carbon atoms; and wherein Z is alternatively selected from the group consisting of -Gly-Z', -Met-Z', -Lys-Z', -Cys-Z', -Gly-Tyr-Z', and -Ala-Tyr-Z', wherein Z' is selected from the group consisting of $-CONH_2$, -COOH, -CONHR, -COOR, $-CONR_2$, $-CH_2OH$, and $-CH_2OR$, wherein R is as defined above; and organic or inorganic addition salts of any of said polypeptides;
wherein the amino acid residue abbreviations used are in accordance with the standard peptide nomenclature:

| | | |
|---|---|---|
| Gly | = | Glycine |
| Tyr | = | L-Tyrosine |
| Ile | = | L-Isoleucine |
| Glu | = | L-Glutamic Acid |
| Thr | = | L-Threonine |
| Phe | = | L-Phenylalanine |
| Ala | = | L-Alanine |
| Lys | = | L-Lysine |
| Asp | = | L-Aspartic Acid |
| Cys | = | L-Cysteine |
| Arg | = | L-Arginine |
| Gln | = | L-Glutamine |
| Pro | = | L-Proline |
| Leu | = | L-Leucine |
| Met | = | L-Methionine |
| Ser | = | L-Serine |
| Asn | = | L-Asparagine |
| His | = | L-Histidine |
| Trp | = | L-Tryptophan |
| Val | = | L-Valine |
| Abu | = | a-Aminobutyric Acid |
| Sar | = | Sarcosine |
| Sar-ol | = | Sarcosine Alcohol |
| DOPA | = | 3,4-Dihydroxyphenylalanine |
| Gly-ol | = | 2-Aminoethanol |
| Hyp | = | trans-4-Hydroxy-L-Proline |
| Met(O) | = | Methionine sulfoxide |
| Met(O)-ol | = | Methionine sulfoxide alcohol |
| Thz | = | L-Thiazolidine-4-carboxylic Acid |
| iLys | = | $N^{\epsilon}$-Isopropyl-L-Lysine |
| 4-Abu | = | 4-Aminobutyric Acid |
| Orn | = | L-Ornithine |
| $D^{\alpha}$Nal | = | $\alpha$-Naphthyl-D-Alanine |
| $D^{\beta}$Nal | = | $\beta$-Naphthyl-D-Alanine |

All three letter amino acid abbreviations preceded by a "D" indicate the D-configuration of the amino acid residue; abbreviations preceded by a "D/L" indicate a mixture of the D- and L-configurations of the designated amino acids; and glycine is included in the scope of the term "naturally occurring L-amino acids".

Group 3 polypeptides are selected from any of the polypeptides having the structure:
Tyr-DArg-Phe-$NH_2$
Tyr-DAla-Phe-$NH_2$;
Tyr-DArg($NO_2$)-Phe-$NH_2$;
Tyr-DAla-(NMe)Phe-Gly-Met(O)-ol;
Tyr-DArg-(NMe)Phe-Gly-Met(O)-ol;
Tyr-DMet(O)-Phe-$NH_2$;

Tyr-DAla-Phe-Gly-$NH_2$;
Tyr-DArg-Phe-Gly-$NH_2$;
Tyr-DThr-Phe-Gly-$NH_2$;
Phe-DArg-Phe-Gly-$NH_2$;
Tyr-DArg-Phe-Sar;
Tyr-DAla-Gly-Phe-$NH_2$;
Tyr-DArg-Gly-Trp-$NH_2$;
Tyr-DArg($NO_2$)-Phe-Gly-$NH_2$;
Tyr-DMet(O)-Phe-Gly-$NH_2$;
(NMe)Tyr-DArg-Phe-Sar-$NH_2$;
Tyr-DArg-Phe-Gly-ol;
Tyr-DArg-Gly-(NMe)Phe-$NH_2$;
Tyr-DArg-Phe-Sar-ol
Tyr-DAla-Phe-Sar-ol
Tyr-DAla-Phe-Gly-Tyr-$NH_2$;
Gly-Tyr-DArg-Phe-Gly-$NH_2$;
Tyr-DThr-Gly-Phe-Thz-$NH_2$;
Gly-Tyr-DAla-Phe-Gly-$NH_2$;
Tyr-DAla-Phe-Gly-ol;
Tyr-DAla-Gly-(NMe)Phe-Gly-ol;
Tyr-DArg-Phe-Sar-$NH_2$;
Tyr-DAla-Phe-Sar-$NH_2$;
Tyr-DAla-Phe-Sar;
Tyr-DAla-Gly-(NMe)Phe-$NH_2$;
Sar-Tyr-DArg-Phe-Sar-$NH_2$;
Tyr-DCys-Phe-Gly-DCys-$NH_2$ (cylic disulfide);
Tyr-DCys-Phe-Gly-DCys-$NH_2$ (free dithiol);
Tyr-DCys-Gly-Phe-DCys-$NH_2$ (cyclic disulfide);
Tyr-DCys-Gly-Phe-DCys-$NH_2$ (free dithiol);
Tyr-DAla-Phe-Gly-Tyr-Pro-Ser-$NH_2$;
Tyr-DAla-Phe-Sar-Tyr-Pro-Ser-$NH_2$;
Tyr-DAla-Phe-Sar-Phe-Pro-Ser-$NH_2$;
Tyr-DAla-Phe-Gly-Tyr-Hyp-Ser-$NH_2$;
Tyr-DAla-Phe-Sar-Tyr-Hyp-Ser-$NH_2$;
Tyr-DAla-Phe-Sar-Phe-Hyp-Ser-$NH_2$;
Tyr-DArg-Phe-Gly-Tyr-Hyp-Ser-$NH_2$;
Tyr-DArg-Phe-Sar-Tyr-Pro-Ser-$NH_2$;
Tyr-DArg-Phe-Sar-Tyr-Hyp-Ser-$NH_2$;
Tyr-DArg-Phe-Gly-Tyr-Pro-Ser-$NH_2$; and
organic or inorganic addition salts of any of said polypeptides of Group 3;

wherein said combination is administered in a ratio such that said combination is effective to cause the synergistic release and elevation of growth hormone in the blood of such animal.

**10.** Combination of Claim 9 wherein said Group 1 polypeptides are selected from any of the polypeptides:
(a) having the following amino acid sequences in Positions 1-44 (numbered from N terminus to C terminus):

(#144) YADAIFTNSYRKVLGQLSARKLLQDIMSRQQGE-SNQERGARARL-X,

(#145) YADAIFTNSYRKVLGQLSARKLLQDIMSRQQGE-RNQEQGARVRL-X,

(#146) YADAIFTNSYRKVLGQLSARKLLQDIMNRQQGE-RNQEQGAKVRL-X,

(#148) YADAIFTNSYRKILGQLSARKLLQDIMNRQQGE-RNQEQGAKVRL-X,

(#149) HADAIFTSSYRRILGQLYARKLLHEIMNRQQGE-RNQEQRSRFN-X;

and functional equivalents thereof:
wherein the C-terminal amino acid has the following truncated general formula

$$-NH-\overset{R'}{\underset{R'}{C}}-$$

wherein each R' independently represents the substituents of the particular amino acid residue, e.g.: hydrogen, alkyl, aryl, amino or acid substituents; X denotes the C terminal end group and is selected from $-CONH_2$, -COOH, -COOR, -CONRR, $-CH_2OH$, and $-CH_2OR$, where R is an alkyl group having 1 to 6 carbon atoms or an aromatic ring having up to 12 carbon atoms; and wherein the amino acid residue abbreviations used are in accordance with the standard peptide nomenclature:

| | | |
|---|---|---|
| G | = | Gly (Glycine), |
| Y | = | Tyr (L-Tyrosine), |
| I | = | Ile (L-Isoleucine), |
| E | = | Glu (L-Glutamic Acid), |
| T | = | Thr (L-Threonine), |
| F | = | Phe (L-Phenylalanine), |
| A | = | Ala (L-Alanine), |
| K | = | Lys (L-Lysine), |
| D | = | Asp (L-Aspartic Acid), |
| C | = | Cys (L-Cysteine), |
| R | = | Arg (L-Arginine), |
| Q | = | Gln (L-Glutamine), |
| P | = | Pro (L-Proline), |
| L | = | Leu (L-Leucine), |
| M | = | Met (L-Methionine), |
| S | = | Ser (L-Serine), |
| N | = | Asn (L-Asparagine), |
| H | = | His (L-Histidine), |
| W | = | Trp (L-Tryptophan), and |
| V | = | Val (L-Valine); |
| Aib | = | $\alpha$-Aminoisobutyric Acid |
| Nle | = | Norleucine |
| Sar | = | Sarcosine |
| Sar-ol | = | Sarcosine Alcohol |
| Gly-ol | = | 2-Aminoethanol |
| Met(O) | = | Methionine Sulfoxide |

(b) any one of said (a) polypeptides having the following amino acid substitutions:
Position 1 of (#144-#148) is DTyr or His;

Position 1 of (#149) is Tyr or DHis;
Position 2 of (#144-#149) is (NMe)DAla or Aib or DAla;
Position 3 of (#144-#149) is DAsp;
Position 4 of (#144-#149) is DAla; and
Position 1 + 2 of (#144-#149) is;
$DTyr^1$ + $DAla^2$, $DTyr^1$ + $(NMe)DAla^2$, or $DTyr^1$ + $Aib^2$;
(c) any one of said (a) or (b) polypeptides having a substitution of Nle for Met at Position 27;
(d) any one of said (a), (b) or (c) polypeptides in which the N-terminus $-NH_2$ is replaced by -NHCOR and wherein R is an alkyl group having 1 to 6 carbon atoms, or an aromatic ring having up to 12 carbon atoms;
(e) fragments of any one of said (a), (b), (c) or (d) polypeptides which contain at least the amino acid residues of Positions 1-29;
(f) having the following specific amino acid sequences in Positions 1-29 (numbered from N terminus to C terminus):
YADAIFTNSYRKVLQQLAARKLLQDIMSR-X,
YADAIFTNSYRKVLQQLLARKLLQDIMSR-X,
YSDAIFSNAYRKILQQLLARKLLQDIMQR-X,
YADAIFSNAYRKILQQLLARKLLQDIMQR-X,
YADAIFSSAYRRLLAQLASRRLLQELLAR-X,
YADAIFTNCYRKVLCQLSARKLLQDIMSR-X (linear dithiol), and
YADAIFTNCYRKVLCQLSARKLLQDIMSR-X (cyclic disulfide);
wherein the C-terminal amino acid and X are as defined above; and modification of any one of these group (f) compounds in accordance with the modifications set forth in (b), (c) and (d) above; and
(g) organic or inorganic addition salts of any of said (a), (b), (c), (d), (e) or (f) polypeptides of Group 1.

**11.** Combination of Claim 9 comprising a compound from each of Group 1 polypeptides and Group 2 polypeptides.

**12.** Combination of Claim 9 comprising a compound from each of Group 2 polypeptides and Group 3 polypeptides.

**13.** Combination of Claim 9 comprising a compound from each of Group 1 polypeptides, Group 2 polypeptides and Group 3 polypeptides.

**14.** Method of causing release and elevation of the level of growth hormone in the blood of an animal, comprising administering an effective dose of the combination of Claim 9.

**15.** Method of causing release and elevation of the level of growth hormone in the blood of an animal, comprising administering an effective dose of the combination of Claim 10.

**16.** Method of Claim 14 wherein said combination comprises a compound from each of Group 1 polypeptides and Group 2 polypeptides.

**17.** Method of Claim 14 wherein said combination comprises a compound from each of Group 2 polypeptides and Group 3 polypeptides.

**18.** Method of Claim 14 wherein said combination comprises a compound from each of Group 1 polypeptides, Group 2 polypeptides and Group 3 polypeptides.

**Patentansprüche**

**1.** Ein Polypeptid, das imstande ist, die Hormonfreisetzung und Hormonspiegelerhöhung von Wachstumshormonen im Blut von Empfängertieren zu fördern, wobei das genannte Polypeptid ausgewählt ist aus der Gruppe, die sich zusammensetzt aus durch die generische Struktur
Ala-AA2-AA3-Trp-AA5-Y-Z
definierten Polypeptiden, worin AA2 aus der Gruppe ausgewählt ist, die von DPhe, DTrp, 5-Fluor-D oder D/LTrp; 6-Fluor-D oder D/LTrp (d.h. mit dem Indolring in 5- oder 6-Stellung fluoriert), (Formyl)DTrp

(d.h. am Indolstickstoff formyliertes DTrp), *XTrp, wobei *XTrp aus der Gruppe der N-monomethylierten DTrp-Isomere (d.h. ($N^{\alpha}$Me)DTrp und (Indol-NMe)DTrp) ausgewählt ist, $D^{\alpha}$Nal und $D^{\beta}$Nal gebildet wird;
AA3 aus der aus Ala, Gly und Ser bestehenden Gruppe ausgewählt ist;
AA5 aus der aus DPhe und (NMe)DPhe bestehenden Gruppe ausgewählt ist;
Y aus der Gruppe ausgewählt ist, die sich zusammensetzt aus

(a) AA7, wobei AA7 aus der Gruppe ausgewählt ist, die aus Arg, iLys, Lys und Orn besteht, und
(b) -AA6-AA7, wobei AA6 aus der Gruppe ausgewählt ist, die alle natürlich vorkommenden L-Aminosäuren, Dipeptide der natürlich vorkommenden L-Aminosäuren wie zum Beispiel Ala-Ala und Verbindungen der Formel

$H_2N(CH_2)_n$-$CO_2H$

mit n = 1 bis 12
umfasst, und AA7 wie oben definiert ist; und

Z die C-endständige Gruppe des genannten Polypeptids oder die C-endständige(n) Aminosäure(n) mit Endgruppe darstellt, wobei Z aus der von -$CONH_2$, -COOH, -COOR, -CONHR, -$CONR_2$, -$CH_2OH$ und -$CH_2OR$ gebildeten Gruppe ausgewählt ist, in der R eine Alkylgruppe mit ein bis sechs Kohlenstoffatomen oder ein aromatischer Ring mit bis zu 12 Kohlenstoffatomen ist, oder wo Z umgekehrt aus der aus -Gly-Z', -Met-Z'. -Lys-Z', -Cys-Z', -Gly-Tyr-Z' und -Ala-Tyr-Z' gebildeten Gruppe ausgewählt ist, worin Z' aus der aus -$CONH_2$, -COOH, -CONHR, -COOR, -$CONR_2$, -$CH_2OH$ und -$CH_2OR$ gebildeten Gruppe ausgewählt ist, in der R wie oben definiert ist;
und aus organischen oder anorganischen salzförmigen Anlagerungsverbindungen jedes beliebigen der genannten Polypeptide;
wobei die für die Aminosäurereste verwendeten Abkürzungen mit der standardisierten Peptidnomenklatur übereinstimmen:

| | |
|---|---|
| Gly | = Glycin |
| Tyr | = L-Tyrosin |
| Ile | = L-Isoleucin |
| Glu | = L-Glutaminsäure |
| Thr | = L-Threonin |
| Phe | = L-Phenylalanin |
| Ala | = L-Alanin |
| Lys | = L-Lysin |
| Asp | = L-Asparaginsäure |
| Cys | = L-Cystein |
| Arg | = L-Arginin |
| Gln | = L-Glutamin |
| Pro | = L-Prolin |
| Leu | = L-Leucin |
| Met | = L-Methionin |
| Ser | = L-Serin |
| Asn | = L-Asparagin |
| His | = L-Histidin |
| Trp | = L-Tryptophan |
| Val | = L-Valin |
| DOPA | = 3,4-Dihydroxyphenylalanin |
| Met(O) | = Methioninsulfoxid |
| Abu | = $\alpha$-Aminobuttersäure |
| iLys | = L-$N^{\epsilon}$-Isopropyllysin |
| 4-Abu | = 4-Aminobuttersäure |
| Orn | = L-Ornithin |
| $D^{\alpha}$Nal | = D-$\alpha$-Naphthylalanin |
| $D^{\beta}$Nal | = D-$\beta$-Naphthylalanin |

Alle dreibuchstabigen Aminosäureabkürzungen, denen ein "D" vorangeht, bezeichnen die D-Konfiguration des Aminosäurerests, während Abkürzungen, denen ein "D/L" vorangeht, Mischungen der D- und L-Konfiguration der betreffenden Aminosäuren bezeichnen; Glycin ist in den Begriff von "natürlich vorkommenden L-Aminosäuren" einbezogen.

2. Ein Polypeptid nach Anspruch 1, wobei das genannte Polypeptid ausgewählt ist aus der aus
   Ala-AA2-Ala-Trp-AA5-AA7-$NH_2$,
   Ala-AA2-Ala-Trp-AA5-AA6-AA7-$NH_2$
   und organischen oder anorganischen salzförmigen Anlagerungsverbindungen jedes beliebigen der genannten Polypeptide bestehenden Gruppe, wobei AA2, AA5, AA6 und AA7 wie oben definiert sind.

3. Ein Polypeptid nach Anspruch 1, wobei das genannte Polypeptid die Sequenz
   Ala-AA2-Ala-Trp-AA5-AA7-$NH_2$
   hat, oder dessen organische oder anorganische salzförmige Anlagerungsverbindungen, wobei AA2, AA5 und AA7 wie oben definiert sind.

4. Ein Polypeptid nach Anspruch 1, wobei das genannte Polypeptid die Sequenz
   Ala-DTrp-Ala-Trp-DPhe-Lys-$NH_2$
   hat, oder dessen organische oder anorganische salzförmige Anlagerungsverbindungen.

5. Eine Methode, die Hormonfreisetzung und Hormonspiegelerhöhung von Wachstumshormonen im Blut von Tieren zu fördern, indem diesen eine wirksame Menge mindestens eines der in Anspruch 1 aufgeführten Polypeptide verabreicht wird.

6. Eine Methode, die Hormonfreisetzung und Hormonspiegelerhöhung von Wachstumshormonen im Blut von Tieren zu fördern, indem diesen eine wirksame Menge mindestens eines der in Anspruch 2 dargelegten Polypeptide verabreicht wird.

7. Eine Methode, die Hormonfreisetzung und Hormonspiegelerhöhung von Wachstumshormonen im Blut von Tieren zu fördern, indem diesen eine wirksame Menge mindestens eines der in Anspruch 3 dargelegten Polypeptide verabreicht wird.

8. Eine Methode, die Hormonfreisetzung und Hormonspiegelerhöhung von Wachstumshormonen im Blut von Tieren zu fördern, indem diesen eine wirksame Menge mindestens eines der in Anspruch 4 dargelegten Polypeptide verabreicht wird.

9. Eine Zusammensetzung, die die Hormonfreisetzung und Hormonspiegelerhöhung von Wachstumshormon im Blut eines Tieres bewirken kann, bestehend aus einer wirksamen Menge von Polypeptiden, die aus Polypeptiden einer zweiten Gruppe von Polypeptiden und mindestens einem Polypeptid einer ersten oder dritten Gruppe ausgewählt sind, wobei die Polypeptide der ersten Gruppe unter beliebigen, natürlich vorkommenden, wachstumshormonfreisetzenden Hormonen und diesen funktionell gleichwertigen Substanzen ausgewählt sind und die genannten Polypeptide auf den wachstumshormonfreisetzenden Hormonrezeptor von Säugetieren und anderen Wirbeltieren sowie Krustentieren wirken;
   die Polypeptide der zweiten Gruppe unter beliebigen Polypeptiden der Struktur
   Ala-AA2-AA3-Trp-AA5-Y-Z
   ausgewählt sind, worin AA2 aus der Gruppe ausgewählt ist, die von DPhe, DTrp, 5-Fluor-D oder D/LTrp; 6-Fluor-D oder D/LTrp (d.h. mit dem Indolring in 5- oder 6-Stellung fluoriert), (Formyl)DTrp (d.h. am Indolstickstoff formyliertes DTrp], *XTrp, wobei *XTrp aus der Gruppe der N-monomethylierten DTrp-Isomere (d.h. ($N^\alpha$Me)DTrp und (Indol-NMe)DTrp) ausgewählt ist, $D^\alpha$Nal und $D^\beta$Nal gebildet wird;
   AA3 aus der aus Ala, Gly und Ser bestehenden Gruppe ausgewählt ist;
   AA5 aus der aus DPhe und (NMe)DPhe bestehenden Gruppe ausgewählt ist;
   Y aus der Gruppe ausgewählt ist, die sich zusammensetzt aus
   (a) AA7, wobei AA7 aus der Gruppe ausgewählt ist, die aus Arg, iLys, Lys und Orn besteht, und
   (b) -AA6-AA7, wobei AA6 aus der Gruppe ausgewählt ist, die alle natürlich vorkommenden L-Aminosäuren, Dipeptide der natürlich vorkommenden L-Aminosäuren wie zum Beispiel Ala-Ala und Verbindungen der Formel

   $$H_2N-(CH_2)_n-CO_2H$$

   mit n = 1 bis 12
   umfasst, und AA7 wie oben definiert ist; und
   Z die C-endständige Gruppe des genannten Polypeptids oder die C-endständige(n) Aminosäure(n) mit Endgruppe darstellt, wobei Z aus der von $-CONH_2$, -COOH, -COOR, -CONHR, $-CONR_2$, $-CH_2OH$ und

$-CH_2OR$ gebildeten Gruppe ausgewählt ist, in der R eine Alkylgruppe mit ein bis sechs Kohlenstoffatomen oder ein aromatischer Ring mit bis zu 12 Kohlenstoffatomen ist, oder wo Z umgekehrt aus der aus -Gly-Z', -Met-Z', -Lys-Z', -Cys-Z', -Gly-Tyr-Z' und -Ala-Tyr-Z' gebildeten Gruppe ausgewählt ist, worin Z' aus der aus $-CONH_2$, -COOH, -CONHR, -COOR, $-CONR_2$, $-CH_2OH$ und $-CH_2OR$ gebildeten Gruppe ausgewählt ist, in der R wie oben definiert ist;
und aus organischen oder anorganischen salzförmigen Anlagerungsverbindungen jedes beliebigen der genannten Polypeptide;
wobei die für die Aminosäurereste verwendeten Abkürzungen mit der standardisierten Peptidnomenklatur übereinstimmen:

| | |
|---|---|
| Gly | = Glycin |
| Tyr | = L-Tyrosin |
| Ile | = L-Isoleucin |
| Glu | = L-Glutaminsäure |
| Thr | = L-Threonin |
| Phe | = L-Phenylalanin |
| Ala | = L-Alanin |
| Lys | = L-Lysin |
| Asp | = L-Asparaginsäure |
| Cys | = L-Cystein |
| Arg | = L-Arginin |
| Gln | = L-Glutamin |
| Pro | = L-Prolin |
| Leu | = L-Leucin |
| Met | = L-Methionin |
| Ser | = L-Serin |
| Asn | = L-Asparagin |
| His | = L-Histidin |
| Trp | = L-Tryptophan |
| Val | = L-Valin |
| Abu | = $\alpha$-Aminobuttersäure |
| Sar | = Sarcosin |
| Sar-ol | = Sarcosinol (Sarcosin Alkohol) |
| DOPA | = 3,4-Dihydroxyphenylalanin |
| Gly-ol | = 2-Aminoethanol |
| Hyp | = L-trans-4-Hydroxyprolin |
| Met(O) | = Methioninsulfoxid |
| Met(O)-ol | = Methioninolsulfoxid (Methioninsulfoxid Alkohol) |
| Thz | = L-Thiazolidin-4-carbonsäure |
| iLys | = L-$N^{\epsilon}$-Isopropyllysin |
| 4-Abu | = 4-Aminobuttersäure |
| Orn | = L-Ornithin |
| $D^{\alpha}$Nal | = D-$\alpha$-Naphthylalanin |
| $D^{\beta}$Nal | = D-$\beta$-Naphthylalanin |

Alle dreibuchstabigen Aminosäureabkürzungen, denen ein "D" vorangeht, bezeichnen die D-Konfiguration des Aminosäurerests, während Abkürzungen, denen ein "D/L" vorangeht, Mischungen der D- und L-Konfiguration der betreffenden Aminosäuren bezeichnen; Glycin ist in den Begriff von "natürlich vorkommenden L-Aminosäuren" einbezogen.
Polypeptide der dritten Gruppe sind ausgewählt unter beliebigen Polypeptiden der Struktur

Tyr-DArg-Phe-$NH_2$
Tyr-DAla-Phe-$NH_2$;
Tyr-DArg($NO_2$)-Phe-$NH_2$;
Try-DAla-(NMe)Phe-Gly-Met(O)-ol;
Tyr-DArg-(NMe)Phe-Gly-Met(O)-ol;
Tyr-DMet(O)-Phe-$NH_2$;
Tyr-DAla-Phe-Gly-$NH_2$;
Tyr-DArg-Phe-Gly-$NH_2$;
Tyr-DThr-Phe-Gly-$NH_2$;
Phe-DArg-Phe-Gly-$NH_2$;

Tyr-DArg-Phe-Sar;
Tyr-DAla-Gly-Phe-$NH_2$;
Tyr-DArg-Gly-Trp-$NH_2$;
Tyr-DArg($NO_2$)-Phe-Gly-$NH_2$;
Tyr-DMet(O)-Phe-Gly-$NH_2$;
(NMe)Tyr-DArg-Phe-Sar-$NH_2$;
Tyr-DArg-Phe-Gly-ol;
Tyr-DArg-Gly-(NMe)Phe-$NH_2$;
Tyr-DArg-Phe-Sar-ol;
Tyr-DAla-Phe-Sar-ol;
Tyr-DAla-Phe-Gly-Tyr-$NH_2$;
Gly-Tyr-DArg-Phe-Gly-$NH_2$;
Tyr-DThr-Gly-Phe-Thz-$NH_2$;
Gly-Tyr-DAla-Phe-Gly-$NH_2$;
Tyr-DAla-Phe-Gly-ol;
Tyr-DAla-Gly-(NMe)Phe-Gly-ol;
Tyr-DArg-Phe-Sar-$NH_2$;
Tyr-DAla-Phe-Sar-$NH_2$;
Tyr-DAla-Phe-Sar;
Tyr-DAla-Gly-(NMe)Phe-$NH_2$;
Sar-Tyr-DArg-Phe-Sar-$NH_2$;
Tyr-DCys-Phe-Gly-DCys-$NH_2$ (zyklisches Disulfid);
Tyr-DCys-Phe-Gly-DCys-$NH_2$ (freies Dithiol);
Tyr-DCys-Gly-Phe-DCys-$NH_2$ (zyklisches Disulfid);
Tyr-DCys-Gly-Phe-DCys-$NH_2$ (freies Dithiol);
Tyr-DAla-Phe-Gly-Tyr-Pro-Ser-$NH_2$;
Tyr-DAla-Phe-Sar-Tyr-Pro-Ser-$NH_2$;
Tyr-DAla-Phe-Sar-Phe-Pro-Ser-$NH_2$;
Tyr-DAla-Phe-Gly-Tyr-Hyp-Ser-$NH_2$;
Tyr-DAla-Phe-Sar-Tyr-Hyp-Ser-$NH_2$;
Tyr-DAla-Phe-Sar-Phe-Hyp-Ser-$NH_2$;
Tyr-DArg-Phe-Gly-Tyr-Hyp-Ser-$NH_2$;
Tyr-DArg-Phe-Sar-Tyr-Pro-Ser-$NH_2$;
Tyr-DArg-Phe-Sar-Tyr-Hyp-Ser-$NH_2$;
Tyr-DArg-Phe-Gly-Tyr-Pro-Ser-$NH_2$; und

organischen oder anorganischen salzförmigen Anlagerungsverbindungen beliebiger Polypeptide der dritten Gruppe;

wobei die genannte Zusammensetzung in einem solchen Verhältnis verabreicht wird, dass sie die synergistische Hormonfreisetzung und Hormonspiegelerhöhung von Wachstumshormon im Blut eines solchen Tieres bewirkt.

**10.** Zusammensetzung gemäss Anspruch 9, worin the Polypeptide der genannten ersten Gruppe ausgewählt sind unter beliebigen Polypeptiden

(a) mit den folgenden Aminosäuresequenzen in Positionen 1 bis 44 (vom N-Ende zum C-Ende gezählt):

(#144) YADAIFTNSYRKVLGQLSARKLLQDIMSRQQGESNQERGARARL-X,
(#145) YADAIFTNSYRKVLGQLSARKLLQDIMSRQQGERNQEQGARVRL-X,
(#146) YADAIFTNSYRKVLGQLSARKLLQDIMNRQQGERNQEQGAKVRL-X,
(#148) YADAIFTNSYRKILGQLSARKLLQDIMNRQQGERNQEQGAKVRL-X,
(#149) HADAIFTSSYRRILGQLYARKLLHEIMNRQQGERNQEQRSRFN-X;

und diesen funktionell gleichwertige Substanzen,

wobei die am C-Ende befindliche Aminosäure die folgende abgekürzte allgemeine Formel hat:

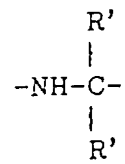

worin jedes der R' unabhängig die Substituenten eines spezifischen Aminosäurerestes darstellt, zum Beispiel Wasserstoff-, Alkyl-, Aryl-, Amino- oder Säuresubstituenten; X bezeichnet die C-endständige Gruppe und ist unter $-CONH_2$, -COOH, -COOR, -CONRR, $-CH_2OH$ und $-CH_2OR$ ausgewählt, wobei R eine Alkylgruppe mit ein bis sechs Kohlenstoffatomen oder ein aromatischer Ring mit bis zu 12 Kohlenstoffatomen ist, und worin die für die Aminosäurereste verwendeten Abkürzungen mit der standardisierten Peptidnomenklatur übereinstimmen:

| | |
|---|---|
| G | = Gly (Glycin), |
| Y | = Tyr (L-Tyrosin), |
| I | = Ile (L-Isoleucin), |
| E | = Glu (L-Glutaminsäure), |
| T | = Thr (L-Threonin), |
| F | = Phe (L-Phenylalanin), |
| A | = Ala (L-Alanin), |
| K | = Lys (L-Lysin), |
| D | = Asp (L-Asparaginsäure), |
| C | = Cys (L-Cystein), |
| R | = Arg (L-Arginin), |
| Q | = Gln (L-Glutamin), |
| P | = Pro (L-Prolin), |
| L | = Leu (L-Leucin), |
| M | = Met (L-Methionin), |
| S | = Ser (L-Serin), |
| N | = Asn (L-Asparagin), |
| H | = His (L-Histidin), |
| W | = Trp (L-Tryptophan) und |
| V | = Val (L-Valin); |
| Aib | = $\alpha$-Aminoisobuttersäure |
| Nle | = Norleucin |
| Sar | = Sarcosin |
| Sar-ol | = Sarcosinol (Sarcosin Alkohol) |
| Gly-ol | = 2-Aminoethanol |
| Met(O) | = Methioninsulfoxid |

(b) unter beliebigen unter (a) genannten Polypeptiden mit den folgenden Aminosäuresubstitutionen:
Position 1 in (#144-#148) ist DTyr oder His;
Position 1 in (#149) ist Tyr oder DHis;
Position 2 in (#144-#149) ist (NMe)DAla oder Aib oder DAla;
Position 3 in (#144-#149) ist DAsp;
Position 4 in (#144-#149) ist DAla; und
Positionen 1 und 2 in (#144-#149) sind $DTyr^1$ + $DAla^2$, $DTyr^1$ + $(NMe)DAla^2$ oder $DTyr^1$ + $Aib^2$;
(c) unter beliebigen der unter (a) oder (b) genannten Polypeptide mit Nle anstatt Met in Position 27;
(d) unter beliebigen der unter (a), (b) oder (c) genannten Polypeptide, in denen das N-endständige $-NH_2$ durch -NHCOR ersetzt ist und worin R eine Alkylgruppe mit ein bis sechs Kohlenstoffatomen oder ein aromatischer Ring mit bis zu 12 Kohlenstoffatomen ist;
(e) unter Bruchstücken eines beliebigen der unter (a), (b), (c) oder (d) genannten Polypeptide mit wenigstens den Aminosäureresten in Positionen 1-29;
(f) mit den folgenden spezifischen Aminosäuresequenzen in Positionen 1-29 (vom N-Ende zum C-Ende gezählt):

YADAIFTNSYRKVLQQLAARKLLQDIMSR-X
YADAIFTNSYRKVLQQLLARKLLQDIMSR-X
YSDAIFSNAYRKILQQLLARKLLQDIMQR-X
YADAIFSNAYRKILQQLLARKLLQDIMQR-X

YADAIFSSAYRRLLAQLASRRLLQELLAR-X
YADAIFTNCYRKVLCQLSARKLLQDIMSR-X (lineares Dithiol) und
YADAIFTNCYRKVLCQLSARKLLQDIMSR-X (zyklisches Disulfid);

worin die C-endständige Aminosäure und X wie oben definiert sind; und mit Abwandlung jeder beliebigen Verbindung der Gruppe (f) gemäss den in (b), (c) und (d) weiter oben dargelegten Abwandlungen; und

(g) unter organischen oder anorganischen salzförmigen Anlagerungsverbindungen jedes beliebigen der unter (a), (b), (c), (d), (e) oder (f) genannten Polypeptide der ersten Gruppe.

**11.** Zusammensetzung gemäss Anspruch 9 mit je einer Verbindung aus der ersten und zweiten Gruppe von Polypeptiden.

**12.** Zusammensetzung gemäss Anspruch 9 mit je einer Verbindung aus der zweiten und dritten Gruppe von Polypeptiden.

**13.** Zusammensetzung gemäss Anspruch 9 mit je einer Verbindung aus der ersten, zweiten und dritten Gruppe von Polypeptiden.

**14.** Verfahren, um die Hormonfreisetzung und Hormonspiegelerhöhung von Wachstumshormon im Blut eines Tieres zu bewirken, indem eine wirksame Dosis der Zusammensetzung nach Anspruch 9 verabreicht wird.

**15.** Verfahren, um die Hormonfreisetzung und Hormonspiegelerhöhung von Wachstumshormon im Blut eines Tieres zu bewirken, indem eine wirksame Dosis der Zusammensetzung nach Anspruch 10 verabreicht wird.

**16.** Verfahren nach Anspruch 14, worin die genannte Zusammensetzung je eine Verbindung aus der ersten und zweiten Gruppe von Polypeptiden umfasst.

**17.** Verfahren nach Anspruch 14, worin die genannte Zusammensetzung je eine Verbindung aus der zweiten und dritten Gruppe von Polypeptiden umfasst.

**18.** Verfahren nach Anspruch 14, worin die genannte Zusammensetzung je eine Verbindung aus der ersten, zweiten und dritten Gruppe von Polypeptiden umfasst.

## Revendications

**1.** Polypeptide capable de favoriser la libération et l'augmentation du niveau sanguin de l'hormone de croissance chez l'animal traité, lequel polypeptide est choisi dans le groupe constitué des polypeptides définis par la formule générale :

Ala-AA2-AA3-Trp-AA5-Y-Z,

où AA2 est choisi dans le groupe constitué par DPhe, DTrp, 5-fluoro-D ou D/LTrp; 6-fluoro-D ou D/LTrp (c'est-à-dire où le noyau indole est fluoré dans la position 5 ou 6), (formyl)DTrp (c'est-à-dire un DTrp portant un groupe formyle sur l'azote de l'indole); *XTrp, où *XTrp est choisi dans le groupe constitué par les isomères N-monométhylés du DTrp (c'est-à-dire ($N^{\alpha}$Me)DTrp et (indole NMe)DTrp), $D^{\alpha}$Nal et $D^{\beta}$Nal;

AA3 est choisi dans le groupe constitué par Ala, Gly et Ser;

AA5 est choisi dans le groupe constitué par DPhe et (NMe)DPhe;

Y est choisi dans le groupe constitué par :

(a) AA7, où AA7 est choisi dans le groupe constitué par Alg, iLys, Lys, et Orn; et

(b) -AA6-AA7, où AA6 est choisi dans le groupe constitué par tous les acides aminés naturels à configuration L et les dipeptides des acides aminés naturels à configuration L, comme par exemple Ala-Ala et les composés de la formule :

$NH_2-(CH_2)_n-CO_2H$,

où n = 1-12,

et où AA7 est comme défini ci-dessus; et

Z représente le groupe C-terminal dudit polypeptide ou l'acide ou les acides aminés C-terminaux plus le groupe terminal, où Z est choisi parmi les groupes $-CONH_2$, -COOH, -COOR, -CONHR, $-CONR_2$, $-CH_2OH$ et $-CH_2OR$, où R est un groupe alkyle ayant 1-6 atomes de carbone ou un noyau aromatique ayant jusqu'à 12 atomes de carbone ; et où Z peut également être choisi parmi les groupes -Gly-Z', -Met-Z', -Lys-Z', Cys-Z', -Gly-Tyr-Z' et -Ala-Tyr-Z', où Z' est choisi parmi les groupes $-CONH_2$, -COOH, -CONHR, -COOR, $-CONR_2$, $-CH_2OH$ et $-CH_2OR$, où R est comme défini ci-dessus;
et les sels d'addition organiques ou minéraux d'un quelconque desdits polypeptides;
où les abréviations utilisées pour les groupes acides aminés sont conformes à la nomenclature conventionnelle :

| | | |
|---|---|---|
| Gly | = | glycine |
| Tyr | = | L-tyrosine |
| Ile | = | L-isoleucine |
| Glu | = | acide L-glutamique |
| Thr | = | L-thréonine |
| Phe | = | L-phénylalanine |
| Ala | = | L-alanine |
| Lys | = | L-lysine |
| Asp | = | acide L-aspartique |
| Cys | = | L-cystéine |
| Arg | = | L-arginine |
| Gln | = | L-glutamine |
| Pro | = | L-proline |
| Leu | = | L-leucine |
| Met | = | L-méthionine |
| Ser | = | L-sérine |
| Asn | = | L-asparagine |
| His | = | L-histidine |
| Trp | = | L-tryptophane |
| Val | = | L-valine |
| DOPA | = | 3,4-dihydroxyphénylalanine |
| Met(O) | = | méthionine sulfoxyde |
| Abu | = | acide $\alpha$-aminobutyrique |
| iLys | = | $N^{\epsilon}$-isopropyl-L-lysine |
| 4-Abu | = | acide 4-aminobutyrique |
| Orn | = | L-ornithine |
| $D^{\alpha}$Nal | = | $\alpha$-naphtyl-D-alanine |
| $D^{\beta}$Nal | = | $\beta$-naphtyl-D-alanine |

Toutes les abréviations d'acides aminés à trois lettres précédées par un "D" indiquent une configuration D du groupe acide aminé; les abréviations précédées par "D/L" indiquent un mélange de configurations D et L des acides aminés indiqués; la glycine est incluse dans le groupe des "acides aminés naturels à configuration L".

**2.** Polypeptide selon la revendication 1, où ledit polypeptide est choisi dans le groupe constitué par :
Ala-AA2-Ala-Trp-AA5-AA7-$NH_2$,
Ala-AA2-Ala-Trp-AA5-AA6-AA7-$NH_2$,
et
les sels d'addition organiques ou minéraux d'un quelconque desdits polypeptides; où AA2, AA5, AA6 et AA7 sont comme défini ci-dessus.

**3.** Polypeptide selon la revendication 1, où ledit polypeptide a la séquence :
Ala-AA2-Ala-Trp-AA5-AA7-$NH_2$, ou
les sels d'addition organiques ou minéraux de celui-ci, où AA2, AA5 et AA7 sont comme défini ci-dessus.

**4.** Polypeptide selon la revendication 1, où ledit polypeptide a la séquence :
Ala-DTrp-Ala-Trp-DPhe-Lys-$NH_2$; ou
les sels d'addition organiques ou minéraux de celui-ci.

**5.** Méthode pour favoriser la libération et l'augmentation du niveau sanguin de l'hormone de croissance chez les animaux en leur administrant une quantité efficace d'au moins un des polypeptides définis dans la revendication 1.

**6.** Méthode pour favoriser la libération et l'augmentation du niveau sanguin de l'hormone de croissance chez les animaux en leur administrant une quantité efficace d'au moins un des polypeptides définis dans la revendication 2.

**7.** Méthode pour favoriser la libération et l'augmentation du niveau sanguin de l'hormone de croissance chez les animaux en leur administrant une quantité efficace d'au moins un des polypeptides définis dans la revendication 3.

**8.** Méthode pour favoriser la libération et l'augmentation du niveau sanguin de l'hormone de croissance chez les animaux en leur administrant une quantité efficace d'au moins un des polypeptides définis dans la revendication 4.

**9.** Combinaison efficace pour provoquer la libération et l'augmentation du niveau sanguin de l'hormone de croissance chez un animal, la combinaison comprenant une quantité efficace de polypeptides choisis parmi les polypeptides du groupe 2 et au moins un polypeptide du groupe 1 ou du groupe 3,
où les polypeptides du groupe 1 sont choisis parmi l'une quelconque des hormones naturelles de libération de l'hormone de croissance et leurs équivalents fonctionnels, où lesdits peptides agissent au niveau des récepteurs de l'hormone de libération de l'hormone de croissance chez les mammifères et autres vertébrés, ainsi que chez les crustacés;
les polypeptides du groupe 2 sont choisis parmi un quelconque des polypeptides ayant la structure :
Ala-AA2-AA3-Trp-AA5-Y-Z,
où AA2 est choisi dans le groupe constitué par DPhe, DTrp, 5-fluoro-D ou D/LTrp; 6-fluoro-D ou D/LTrp (c'est-à-dire où le noyau indole est fluoré en position 5 ou 6), (formyl)DTrp (c'est-à-dire un DTrp portant un groupe formyle sur l'azote de l'indole); *XTrp, où *XTrp est choisi dans le groupe constitué des isomères M-monométhylés du DTrp (c'est-à-dire $(N^{\alpha}Me)$DTrp et (indole NMe)DTrp), $D^{\alpha}$Nal et $D^{\beta}$Nal;
AA3 est choisi dans le groupe constitué par Ala, Gly et Ser;
AA5 est choisi dans le groupe constitué par DPhe et (NMe)DPhe;
Y est choisi dans le groupe constitué par :
(a) AA7, où AA7 est choisi dans le groupe constitué par Arg, iLys, Lys, et Orn; et
(b) -AA6-AA7, où AA6 est choisi dans le groupe constitué par tous les acides aminés naturels à configuration L et les dipeptides des acides aminés naturels à configuration L, comme par exemple Ala-Ala et les composés de la formule

$NH_2$-$(CH_2)_n$-$CO_2H$,

où n = 1-12,
et où AA7 est comme défini ci-dessus; et
Z représente le groupe C-terminal dudit polypeptide ou l'acide ou les acides aminés C-terminaux plus le groupe terminal, où Z est choisi parmi les groupes $-CONH_2$, -COOH, -COOR, -CONHR, $-CONR_2$, $-CH_2OH$ et $-CH_2OR$, où R est un groupe aile ayant 1-6 atomes de carbone ou un noyau aromatique ayant jusqu'à 12 atomes de carbone ; et où Z peut également être choisi parmi les groupes -Gly-Z', -Met-Z', -Lys-Z', Cys-Z', -Gly-Tyr-Z' et -Ala-Tyr-Z', où Z' est choisi parmi les groupes $-CONH_2$, -COOH, -CONHR, -COOR, $-CONR_2$, $-CH_2OH$ et $-CH_2OR$, où R est comme défini ci-dessus;
et les sels d'addition organiques ou minéraux d'un quelconque desdits polypeptides;
où les abréviations utilisées pour les acides aminés sont conformes à la nomenclature conventionnelle :

| | |
|---|---|
| Gly | = glycine |
| Tyr | = L-tyrosine |
| Ile | = L-isoleucine |
| Glu | = acide L-glutamique |
| Thr | = L-thréonine |
| Phe | = L-phénylalanine |
| Ala | = L-alanine |
| Lys | = L-lysine |

Asp = acide L-aspartique
Cys = L-cystéine
Alg = L-arginine
Gln = L-glutamine
Pro = L-proline
Leu = L-leucine
Met = L-méthionine
Ser = L-sérine
Asn = L-asparagine
His = L-histidine
Trp = L-tryptophane
Val = L-valine
Abu = acide $\alpha$-aminobutyrique
Sar = sarcosine
Sar-ol = sarcosine alcool
DOPA = 3,4-dihydroxyphenylalanine
Gly-ol = 2-aminoethanol
Hyp = trans-4-hydroxy-L-proline
Met(O) = methionine sulfoxyde
Met(O)-ol = methionine sulfoxyde alcool
Thz = acide L-thiazolidine-4-carboxylique
iLys = $N^{\epsilon}$-isopropyl-L-lysine
4-Abu = acide 4-aminobutyrique
Orn = L-ornithine
$D^{\alpha}$Nal = $\alpha$-naphtyl-D-alanine
$D^{\beta}$Nal = $\beta$-naphtyl-D-alanine

Toutes les abréviations d'acides aminés à trois lettres précédées par un "D" indiquent une configuration D du groupe acide aminé; les abréviations précédées par "D/L" indiquent un mélange de configurations D et L des acides aminés indiqués; la glycine est incluse dans le groupe des "acides aminés naturels" à configuration L.

Les polypeptides du groupe 3 sont choisis parmi un quelconque des polypeptide ayant la structure :

Tyr-DArg-Phe-$NH_2$;
Tyr-DAla-Phe-$NH_2$;
Tyr-DArg($NO_2$)-Phe-$NH_2$;
Tyr-DAla-(NMe)Phe-Gly-Met(O)-ol;
Tyr-DArg-(NMe)Phe-Gly-Met(O)-ol;
Tyr-DMet(O)-Phe-$NH_2$;
Tyr-DAla-Phe-Gly-$NH_2$;
Tyr-DArg-Phe-Gly-$NH_2$;
Tyr-DThr-Phe-Gly-$NH_2$;
Phe-DArg-Phe-Gly-$NH_2$;
Tyr-DArg-Phe-Sar;
Tyr-DAla-Gly-Phe-$NH_2$;
Tyr-DArg-Gly-Trp-$NH_2$;
Tyr-DArg($NO_2$)-Phe-Gly-$NH_2$;
Tyr-DMet(O)-Phe-Gly-$NH_2$;
(NMe)Tyr-DArg-Phe-Sar-$NH_2$;
Tyr-DArg-Phe-Gly-ol;
Tyr-DArg-Gly-(NMe)Phe-$NH_2$;
Tyr-DArg-Phe-Sar-ol;
Tyr-DAla-Phe-Sar-ol;
Tyr-DAla-Phe-Gly-Tyr-$NH_2$;
Gly-Tyr-DArg-Phe-Gly-$NH_2$;
Tyr-DThr-Gly-Phe-Thz-$NH_2$;
Gly-Tyr-DAla-Phe-Gly-$NH_2$;
Tyr-DAla-Phe-Gly-ol;
Tyr-DAla-Gly-(NMe)Phe-Gly-ol;
Tyr-DArg-Phe-Sar-$NH_2$;

Tyr-DAla-Phe-Sar-$NH_2$;
Tyr-DAla-Phe-Sar;
Tyr-DAla-Gly(NMe)Phe-$NH_2$;
Sar-Tyr-DArg-Phe-Sar-$NH_2$;
Tyr-DCys-Phe-Gly-DCys-$NH_2$ (disulfure cyclique);
Tyr-DCys-Phe-Gly-DCys-$NH_2$ (dithiol libre);
Tyr-DCys-Gly-Phe-DCys-$NH_2$ (disulfure cyclique);
Tyr-DCys-Gly-Phe-DCys-$NH_2$ (dithiol libre);
Tyr-DAla-Phe-Gly-Tyr-Pro-Ser-$NH_2$;
Tyr-DAla-Phe-Sar-Tyr-Pro-Ser-$NH_2$;
Tyr-DAla-Phe-Sar-Phe-Pro-Ser-$NH_2$;
Tyr-DAla-Phe-Gly-Tyr-Hyp-Ser-$NH_2$;
Tyr-DAla-Phe-Sar-Tyr-Hyp-Ser-$NH_2$;
Tyr-DAla-Phe-Sar-Phe-Hyp-Ser-$NH_2$;
Tyr-DArg-Phe-Gly-Tyr-Hyp-Ser-$NH_2$;
Tyr-DArg-Phe-Sar-Tyr-Pro-Ser-$NH_2$;
Tyr-DArg-Phe-Sar-Tyr-Hyp-Ser-$NH_2$;
Tyr-DArg-Phe-Gly-Tyr-Pro-Ser-$NH_2$; et

les sels d'addition organiques ou minéraux d'un quelconque desdits polypeptides du groupe 3;
où ladite combinaison est administrée dans un rapport tel que ladite combinaison soit efficace pour provoquer un effet de synergie sur la libération et l'augmentation du niveau sanguin de l'hormone de croissance chez les animaux susmentionnés.

**10.** Combinaison selon la revendication 9, où lesdits polypeptides du groupe 1 sont choisis parmi n'importe lesquels des polypeptides suivants :
(a) ayant les séquences des acides aminés suivants dans les positions 1-44 (numérotation depuis l'extrémité N terminale jusqu'à l'extrémité C terminale) :

**(#144) YADAIFTNSYRKVLGQLSARKLLQDIMSRQQGESNQERGARARL-X,**
**(#145) YADAIFTNSYRKVLGQLSARKLLQDIMSRQQGERNQEQGARVRL-X,**
**(#146) YADAIFTNSYRKVLGQLSARKLLQDIMNRQQGERNQEQGAKVRL-X,**
**(#148) YADAIFTNSYRKILGQLSARKLLQDIMNRQQGERNQEQGAKVRL-X,**
**(#149) HADAIFTSSYRRILGQLYARKLLHEIMNRQQGERNQEQRSRFN-X;**

et leurs équivalents fonctionnels;
où l'acide aminé C-terminal a la formule générale suivante

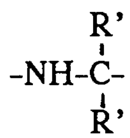

où chaque R' représente d'une manière indépendante les substituants du groupe acide aminé particulier considéré, par exemple : hydrogène, alkyle, aryle, amino ou acide; X représente le groupe C-terminal et il est choisi parmi les groupes -$CONH_2$, COOH, -COOR, -CONRR, -$CH_2OH$, et -$CH_2OR$, où R est un groupe alkyle ayant de 1 à 6 atomes de carbone ou un noyau aromatique ayant jusqu'à 12 atomes de carbone; et où les abréviations utilisées pour les groupes des acides aminés sont conformes à la nomenclature conventionnelle :

| | | |
|---|---|---|
| G | = | Gly (glycine) |
| Y | = | Tyr (L-tyrosine) |
| I | = | Ile (L-isoleucine) |
| E | = | Glu (acide L-glutamique) |
| T | = | Thr (L-thréonine) |
| F | = | Phe (L-phénylalanine) |

| | | |
|---|---|---|
| A | = | Ala (L-alanine) |
| K | = | Lys (L-lysine) |
| D | = | Asp (acide L-aspartique) |
| C | = | Cys (L-cystéine) |
| R | = | Arg (L-arginine) |
| Q | = | Gln (L-glutamine) |
| P | = | Pro (L-proline) |
| L | = | Leu (L-leucine) |
| M | = | Met (L-méthionine) |
| S | = | Ser (L-sérine) |
| N | = | Asn (L-asparagine) |
| H | = | His (L-histidine) |
| W | = | Trp (L-tryptophane), et |
| V | = | Val (L-valine) |
| Aib | = | acide $\alpha$-aminobutyrique |
| Nle | = | norleucine |
| Sar | = | sarcosine |
| Sar-ol | = | sarcosine alcool |
| Gly-ol | = | 2-aminoéthanol |
| Met(O) | = | méthionine sulfoxyde |

(b) un quelconque desdits polypeptides (a) avec les substitutions suivantes d'acides aminés :
la position 1 de (#144-#148) est DTyr ou His;
la position 1 de (#149) est Tyr ou DHis;
la position 2 de (#144-#149) est (NMe)DAla ou Aib ou DAla;
la position 3 de (#144-#149) est DAsp;
la position 4 de (#144-#149) est DAla; et
la position 1 + 2 de (#144-#149) est $DTyr^1 + DAla^2$, $DTyr^1 + (NMe)DAla^2$ ou $DTyr^1 + Aib^2$;
(c) un quelconque desdits polypeptides (a) ou (b), où Met en position 27 est remplacé par Nle;
(d) un quelconque desdits polypeptides (a), (b) ou (c) où le $-NH_2$ N-terminal est remplacé par -NHCOR et où R est un groupe alkyle ayant 1 à 6 atomes de carbone ou un noyau aromatique ayant jusqu'à 12 atomes de carbone;
(e) des fragments d'un quelconque desdits polypeptides (a), (b), (c) ou (d) contenant au moins les groupes acides aminés des positions 1-29;
(f) ayant les séquences particulières suivantes des acides aminés dans les positions 1-29 (numérotation depuis l'extrémité N terminale jusqu'à l'extrémité C-terminale) :

YADAIFTNSYRKVLQQLAARKLLQDIMSR-X,
YADAIFTNSYRKVLQQLLARKLLQDIMSR-X,
YSDAIFSNAYRKILQQLLARKLLQDIMQR-X,
YADAIFSNAYRKILQQLLARKLLQDIMQR-X,
YADAIFSSAYRRLLAQLASRRLLQELLAR-X,
YADAIFTNCYRKVLCQSARKLLQDIMSR-X,
(dithiol linéaire) et
YADAIFTNCYRKVLCQLSARKLLQDIMSR-X (disulfure cyclique);

où l'acide aminé C-terminal et X sont comme défini ci-dessus; et des modifications d'un quelconque des composés de ce groupe (f) conformément aux modifications indiquées en (b), (c) et (d) ci-dessus; et

(g) les sels organiques et minéraux d'un quelconque desdits polypeptides (a), (b), (c), (d), (e) ou (f) du groupe 1.

**11.** Combinaison de la revendication 9 comprenant un composé de chacun des groupes de polypeptides 1 et 2.

**12.** Combinaison de la revendication 9 comprenant un composé de chacun des groupes de polypeptides 2 et 3.

**13.** Combinaison de la revendication 9, comprenant un composé de chacun des groupes de polypeptides 1, 2 et 3.

14. Méthode pour provoquer la libération et l'augmentation du niveau sanguin de l'hormone de croissance chez un animal, comprenant d'administrer une dose efficace de la combinaison de la revendication 9.

15. Méthode pour provoquer la libération et l'augmentation du niveau sanguin de l'hormone de croissance chez un animal, comprenant d'administrer une dose efficace de la combinaison de la revendication 10.

16. Méthode selon la revendication 14, où ladite combinaison comprend un composé de chacun des groupes de polypeptides 1 et 2.

17. Méthode selon la revendication 14, où ladite combinaison comprend un composé de chacun des groupes de polypeptides 2 et 3.

18. Méthode selon la revendication 14, où ladite combinaison comprend un composé de chacun des groupes de polypeptides 1, 2 et 3.